# EUROPEAN PATENT SPECIFICATION

(11) **EP 4 329 618 B1**
(45) Date of publication and mention of the grant of the patent: **17.06.2026**
(21) Application number: 22866704.4
(22) Date of filing: 08.09.2022
(51) Int. Cl.: A61B 6/00, A61B 6/03, A61B 6/04, A61B 8/00, A61B 6/10

(54) **MEDICAL DEVICES, METHODS AND SYSTEMS FOR MONITORING THE MEDICAL DEVICES**
MEDIZINISCHE VORRICHTUNGEN, VERFAHREN UND SYSTEME ZUR ÜBERWACHUNG DER MEDIZINISCHEN VORRICHTUNGEN
DISPOSITIFS MÉDICAUX, PROCÉDÉS ET SYSTÈMES DE SURVEILLANCE DES DISPOSITIFS MÉDICAUX

(30) Priority: 08.09.2021 CN 202111052132; 16.09.2021 CN 202111087938
(43) Date of publication of application: 06.03.2024
(73) Proprietor: Shanghai United Imaging Healthcare Co., Ltd., Shanghai 201807 (CN)
(72) Inventor: SONG, Shujie, Shanghai 201807 (CN); LI, Die, Shanghai 201807 (CN); YANG, Changwei, Shanghai 201807 (CN)
(74) Representative: Wang, Bo
(86) International application number: PCT/CN2022/117823
(87) International publication number: WO 2023/036243

(56) References cited:
- WO-A1-2021/136250
- CN-A- 109 464 757
- CN-A- 109 938 763
- CN-A- 112 515 695
- CN-A- 112 741 643
- CN-A- 113 116 365
- CN-A- 113 647 967
- CN-A- 113 796 875
- US-A1- 2018 061 045
- US-A1- 2021 265 057
- NICOLAI P. ET AL: "A Novel 3D Camera Based Supervision System for Safe Human-Robot Interaction in the Operating Room", vol. 3, 1 October 2015 (2015-10-01), pages 410 - 417, XP055900031, ISSN: 2301-3702, Retrieved from the Internet <URL:http://www.joace.org/uploadfile/2015/0408/20150408114346169.pdf> DOI: 10.12720/joace.3.5.410-417

## Description

### CROSS-REFERENCE TO RELATED APPLICATIONS

This application claims priority to Chinese Patent Application No. 202111052132.5, filed on September 8, 2021, and Chinese Patent Application No. 202111087938.8, filed on September 16, 2021.

### TECHNICAL FIELD

The present disclosure relates to the field of medical apparatus and instruments, in particular, to a medical device, a method and a system for monitoring the medical device.

### BACKGROUND

In the process of treatment and diagnosis for a patient via a medical device, a medical device for scanning is usually required to scan the patient to obtain relevant information of a nidus. However, in the process of scanning, an operator (e.g., a medical staff) needs to adjust the height of an examination table to ensure that an object may easily go to the examination table and/or get out of the examination table, and needs to help/guide the object to adjust a position to ensure that a scanned image contains target position information of the object. And keys of the medical device often get stuck in the process of using the medical device, or other abnormal conditions caused by an unexpected motion of the medical device may occur when there is no operator. In the process of scanning, not only the working intensity of the operator is high, but also the scanning efficiency is low.

Therefore, it is hoped to provide an automatically controlled medical device, a method and a system for monitoring the medical device to improve the scanning efficiency. US2021265057A1 discloses systems and methods for automated healthcare services.

### SUMMARY OF THE DISCLOSURE

The invention is set out in the appended set of claims. One aspect of the present disclosure may provide a method for monitoring a medical device. The method may include: obtaining a plurality of first images of a scanning room acquired by a first monitoring device arranged in the scanning room; obtaining an analysis result by analyzing the plurality of first images; and controlling the medical device based on the analysis result, the medical device including an examination table; wherein a control mode of the medical device may include at least one of controlling the medical device to adjust a height of the examination table, controlling the medical device to send an object to a target position, or controlling a running state of the medical device.

Another aspect of the present disclosure may provide a medical device. The medical device may include: a scanner configured to perform a medical scan to obtain scanning data of a target position of an object; an image acquisition device configured to obtain a plurality of first images of a scanning room; a processor configured to obtain an analysis result by analyzing the plurality of first images and determine a control mode of the medical device based on the analysis result; and a control mechanism configured to control the medical device based on the control mode.

Another aspect of the present disclosure may provide system for monitoring a medical device. The system may include: an acquisition module configured to obtain a plurality of first images of a scanning room acquired by a first monitoring device arranged in the scanning room; an image analysis module configured to obtain an analysis result by analyzing the plurality of first images; and a control module configured to control the medical device based on the analysis result, the medical device including an examination table, wherein a control mode of the medical device includes at least one of controlling the medical device to adjust a height of the examination table, controlling the medical device to send an object to a target position, or controlling a running state of the medical device.

Another aspect of the present disclosure may provide an apparatus for monitoring a medical device. The device may include at least one processor and at least one memory, wherein the at least one memory is configured to store executable instructions, and the at least one processor is configured to perform a method in above embodiments when executing at least a portion of the executable instructions.

Another aspect of the present disclosure may provide a non-transitory computer readable medium that stores executable instructions, wherein when the executable instructions are read by at a computing device, the computing device performs a method in above embodiments.

### BRIEF DESCRIPTION OF THE DRAWINGS

The present disclosure is further illustrated in terms of exemplary embodiments, and these exemplary embodiments are described in detail with reference to the drawings. These embodiments are not restrictive. In these embodiments, the same number indicates the same structure, wherein:
FIG. 1 is a schematic diagram illustrating an application scenario of an exemplary method for monitoring a medical device according to some embodiments of the present disclosure:
FIG. 2 is a schematic diagram illustrating an exemplary software and/or hardware of a computing device according to some embodiments of the present disclosure;
FIG. 3 is a schematic diagram illustrating an exemplary software and/or hardware of a terminal device according to some embodiments of the present disclosure;
FIG. 4 is a block diagram illustrating an exemplary processing device for monitoring a medical device according to some embodiments of the present disclosure;
FIG. 5 is a flowchart illustrating an exemplary process for monitoring a medical device according to some embodiments of the present disclosure;
FIG. 6 is a flowchart illustrating an exemplary process for controlling a medical device according to some embodiments of the present disclosure;
FIG. 7 is a schematic diagram illustrating an exemplary arrangement of structures of a medical device according to some embodiments of the present disclosure;
FIG. 8 is a flowchart illustrating an exemplary process for monitoring a running state of a medical device according to some embodiments of the present disclosure;
FIG. 9 is a flowchart illustrating an exemplary process for obtaining expected running information of a medical device according to some embodiments of the present disclosure; and
FIG. 10 is a schematic diagram illustrating an exemplary system for monitoring a medical device according to some embodiments of the present disclosure.

### DETAILED DESCRIPTION

In order to illustrate the technical solutions related to the embodiments of the present disclosure, brief introduction of the drawings referred to in the description of the embodiments is provided below. Obviously, drawings described below are only some examples or embodiments of the present disclosure. Those having ordinary skills in the art, without further creative efforts, may apply the present disclosure to other similar scenarios according to these drawings. Unless stated otherwise or obvious from the context, the same reference numeral in the drawings refers to the same structure and operation.

It will be understood that the terms "system," "device," "unit," and/or "module" used herein are one method to distinguish different components, elements. parts, sections, or components of different levels in ascending order. However, the terms may be displaced by other expressions if they may achieve the same purpose.

As shown in the present disclosure and claims, unless the context clearly indicates exceptions, the words "a," "an," "one," and/or "the" do not specifically refer to the singular, but may also include the plural. The terms "including" and "comprising" only suggest that the steps and elements that have been clearly identified are included, and these steps and elements do not constitute an exclusive list, and the method or device may also include other steps or elements.

The flowcharts used in the present disclosure may illustrate operations executed by the system according to embodiments in the present disclosure. It should be understood that a previous operation or a subsequent operation of the flowcharts may not be accurately implemented in order. Conversely, various operations may be performed in inverted order, or simultaneously. Moreover, other operations may be added to the flowcharts, and one or more operations may be removed from the flowcharts.

In the process of treatment and diagnosis for a patient via various medical devices, a medical staff may need to operate an examination table of the medical device based on a height, a weight and other information of different patients, and adjust the height of the examination table to an appropriate height, which is convenient for patients to go to or get off the examination table. The medical stuff may also need to guide the patient to adjust a position, to ensure that a scanned image contains information of a target position of the patient. Thus, the workload of the medical staff may be increased, and the scanning efficiency may be reduced. In addition, in the process of using the medical device, various abnormal conditions may occur, such as abnormal motion of the medical device, mistakenly touching from the patient, key being stuck, etc., which may cause potential harm to the patient, and affect the scanning efficiency.

Some embodiments of the present disclosure provide a medical device and a method for monitoring the medical device. A plurality of images of a medical device and/or an object may be obtained by at least one monitoring device in a scanning room. Based on an analysis result of the plurality of images, a height of an examination table of the medical device may be controlled and a running state of the medical device may be monitored. Thus, the time of the scanning may be reduce, and the scanning may be rapidly performed.

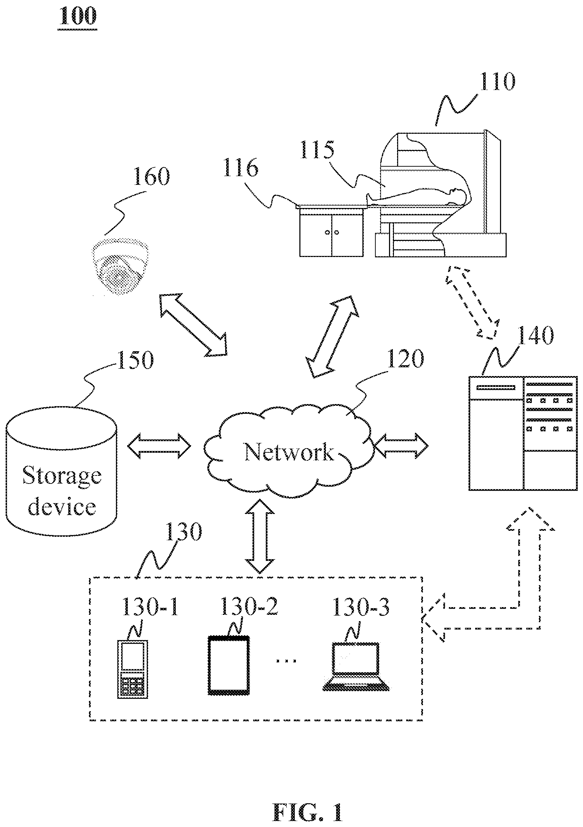

FIG. 1 is a schematic diagram illustrating an application scenario of an exemplary system for monitoring a medical device according to some embodiments of the present disclosure.

As shown in FIG. 1, a monitoring system 100 may include a medical device 110 (e.g., a CT scanner shown in FIG. 1), a network 120, a terminal 130, a processing device 140, a storage device 130, and a monitoring device 160. The components in the monitoring system 100 may be connected with each other in various ways. Merely by way of example, as shown in FIG. 1, the medical device 110 may be connected with the processing device 140 via the network 120. As another example, the medical device 110 may be directly connected with the processing device 140 (as shown by the two-way arrow in the dotted line connecting the medical device 110 and the processing device 140). As still another example, the storage device 150 may be connected with the processing device 140 directly or via the network 120. As still another example, the monitoring device 160 may be connected with the processing device 140 via the network 120. As a further example, the terminal 130 may be directly connected with the processing device 140 (as indicated by the two-way arrow in the dotted line connecting the terminal 130 and the processing device 140) or connected with the processing device 140 via the network 120.

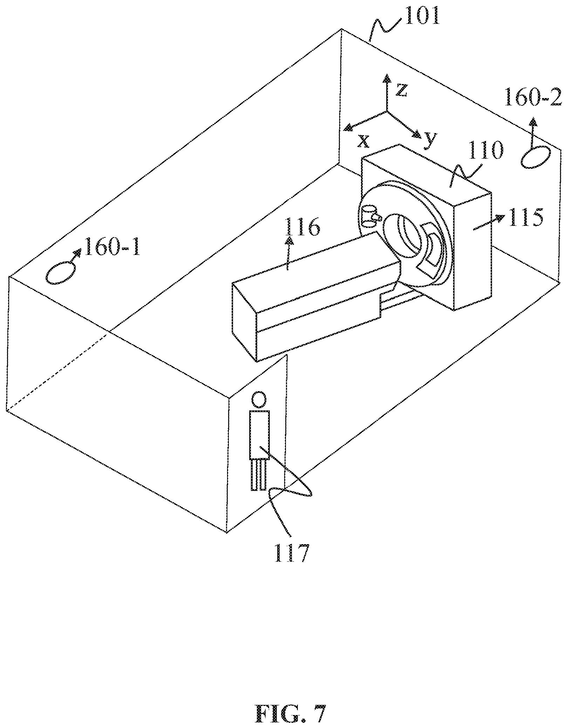

The medical device 110 may scan an object and/or generate a plurality of data about the object. In the present disclosure, the object may also be referred to as a scanning object, a target object, a target, or a detected object, and the above terms may be used interchangeably. In some embodiments, the object may be a creature, such as a patient, an animal, or the like. When the object needs to be scanned, the object may be placed on an examination table 116. And the object may move with the examination table 116 along a transverse direction (e.g., the x-axis direction in FIG. 7), a longitudinal direction (e.g., the z-axis direction in FIG. 7) of the medical device 110, and may enter a scanning region 115. After the object enters the scanning region 115, the medical device 110 may perform an operation based on a scanning protocol, and a corresponding medical image of the object may be obtained.

In some embodiments, the medical device 110 may include a single-mode scanning device and/or a multi-mode scanning device. The single-mode scanning device may include, for example, a computed tomography (CT) device, a positron emission computed tomography (PET) scanning device, a magnetic resonance imaging (MRI) scanning device, an X-ray scanning device, etc. The multi-mode scanning device may include, for example, an X-ray imaging-magnetic resonance imaging (X-ray MRI) scanning device, a positron emission tomography X-ray imaging (PET-X-ray) scanning device, a single photon emission computed tomography magnetic resonance imaging (SPECT-MRI) scanning device, a positron emission tomography computer tomography (PET-CT) scanning device, etc. The scanning device provided above is merely for illustration and is not intended to limit the scope of the present disclosure. As used herein, the term "imaging mode" or "mode" may broadly refer to an imaging method or technology that collects, generates, processes, and/or analyzes the imaging information of a target object.

The network 120 may include any suitable network that facilitates the exchange of information and/or data of the monitoring system 100. In some embodiments, one or more components of the monitoring system 100 (e.g., the medical device 110, the terminal 130, the processing device 140, the storage device 150, or the monitoring device 160) may transmit information and/or data with one or more other components of the monitoring system 100 via network 120. For example, the processing device 140 may obtain a medical image of the object from the medical device 110 via the network 120. In some embodiments, the network 120 may be any one or more of a wired or wireless network. In some embodiments, the network may be point-to-point, shared, central and other topologies or a combination of a plurality of topologies. In some embodiments, the network 120 may include one or more network access points. For example, network 120 may include wired or wireless network access points.

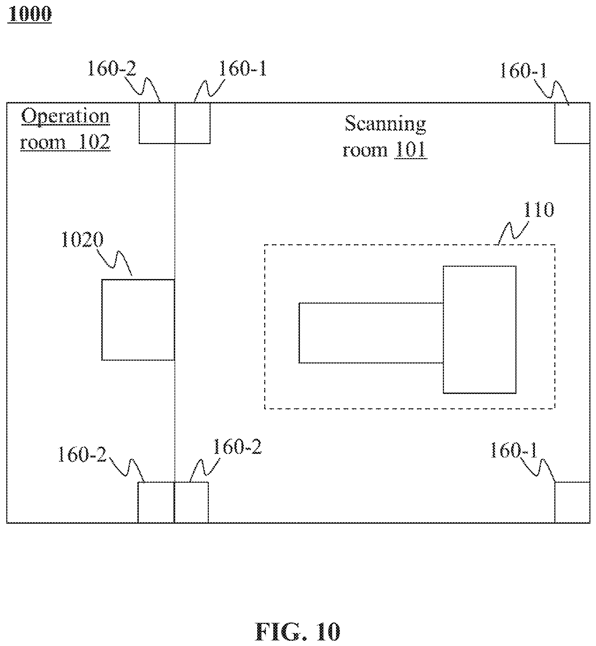

The terminal 130 may include a mobile device 130-1, a tablet computer 130-2, a notebook computer 130-3, or any combination thereof. In some embodiments, the tenninal 130 may interact with other components in the monitoring system 100 via the network 120. For example, the terminal 130 may send one or more control instructions to the medical device 110 to control the examination table 116 to support the object into the scanning region 115. For another example, the terminal 130 may also receive data such as medical images sent by the medical device 110. In some embodiments, the terminal 130 may receive information and/or instructions input by a user (e.g., a user of the medical device 110, such as a doctor) and send the received information and/or instructions to the medical device 110 or the processing device 140 via the network 120. For example, a doctor may input an operation instruction to the medical device 110 via the terminal 130. In some embodiments, the terminal 130 may be a portion of the processing device 140. For example, the terminal 130 and the processing device 140 may be integrated as a control device (e.g., a console 1020 shown in FIG. 10) of the medical device 110. In some embodiments, the terminal 130 may be omitted.

The processing device 140 may process data and/or information obtained from the medical device 110, the terminal 130, the storage device 150, and/or the monitoring device 160. For example, the processing device 140 may acquire a medical image of the object. For another example, the processing device 140 may determine posture information of the object based on a plurality of first images of the object collected by the monitoring device 160, and then adjust the height of the examination table 116. For example, the processing device 140 may determine actual positioning information of the object based on a plurality of second images of the object on the examination table acquired by the monitoring device 160. For another example, the processing device 140 may acquire a video of the medical device 110 based on the monitoring device 160, and determine the actual running information of the medical device 110 by analyzing the video, so as to determine whether the running state of the medical device 110 is abnormal.

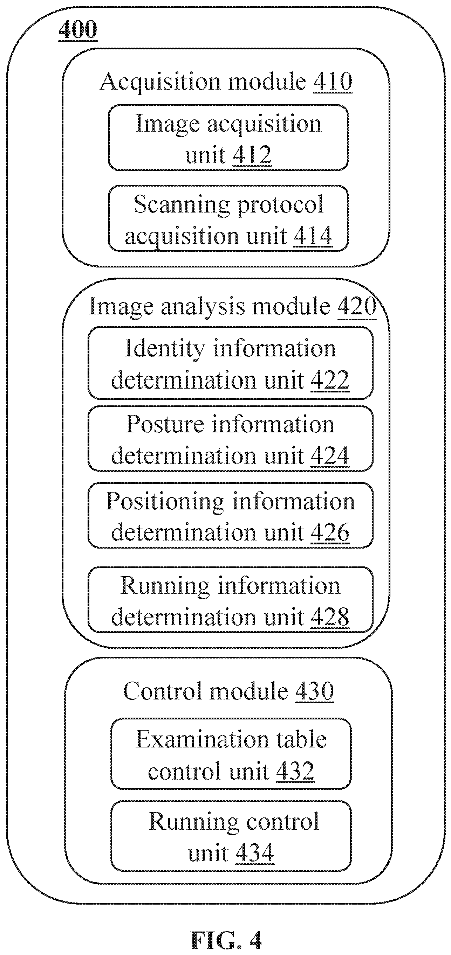

In some embodiments, the processing device 140 may be a single server or a group of servers. The group of servers may be centralized or distributed. In some embodiments, the processing device 140 may be local or remote. For example, the processing device 140 may access information and/or data stored in or acquired by the medical device 110, the terminal 130, and/or the storage device 150 via the network 120. For another example, the processing device 140 may be directly connected with the medical device 110, the terminal 130, the storage device 150, and/or the monitoring device 160 to access stored or acquired information and/or data. In some embodiments, the processing device 140 may be implemented on a cloud platform. Merely by way of example, the cloud platform may include private cloud, public cloud, hybrid cloud, community cloud, distributed cloud, internal cloud, multi- cloud, or the like, or any combination thereof. In some embodiments, the processing device 140 may be implemented on a computing device 200 including one or more components shown in FIG. 4 in this specification.

The storage device 150 may store data and/or instructions. In some embodiments, the storage device 150 may store data obtained from the medical device 110, the terminal 130, and/or the processing device 140. For example, the storage device 150 may store motion information of the object designed in advance by a user (e.g., a doctor, an imaging technician). In some embodiments, the storage device 150 may store data and/or instructions that the processing device 140 may execute or use to execute the exemplary methods described in the present disclosure. For example, the storage device 150 may store instructions of the processing device 140 to execute the methods shown in the flowcharts in the present disclosure. In some embodiments, the storage device 150 may include a mass storage device, a removable storage device, a volatile read-write memory, a read-only memory (ROM), etc., or any combination thereof. In some embodiments, the storage device 150 may be implemented on a cloud platform.

In some embodiments, the storage device 150 may be connected with the network 120 to communicate with one or more components of the monitoring system 100 (e.g., the medical device 110, the terminal 130, the processing device 140, the monitoring device 160, etc.). One or more components of the monitoring system 100 may access data or instructions stored in the storage device 150 via the network 120. In some embodiments, the storage device 150 may be directly connected with or communicate with one or more components of the monitoring system 100 (e.g., the medical device 110, the terminal 130, the processing device 140, the monitoring device 160, etc.). In some embodiments, the storage device 150 may be a portion of the processing device 140.

The monitoring device 160 may be configured to collect images in a scanning room (e.g., a scanning room 101 shown in FIG. 7 or FIG. 10). In some embodiments, the monitoring device 160 may be configured to collect a plurality of first images when the object enters the scanning room. In some embodiments, the monitoring device 160 may be configured to acquire a plurality of second images of the object on the examination table 116. In some embodiments, the monitoring device 160 may be configured to acquire a plurality of third images of the object. In some embodiments, the monitoring device 160 may be configured to acquire key control information of the medical device 110. In some embodiments, the monitoring device 160 may be configured to obtain running state information of the medical device 110. In some embodiments, the monitoring device 160 may be configured to obtain current operation information of the medical device 110.

In some embodiments, the monitoring device 160 may be configured to acquire image information of the object and image information of the medical device, respectively. For example, the monitoring device 160 may first acquire the first image and the second image of the object, and then acquire the current operation information of the medical device 110. As another example, the monitoring device 160 may first acquire the first image of the object, then acquire the current operation information of the medical device 110, and finally acquire the second image of the object. In some embodiments, the monitoring device 160 may be configured to simultaneously acquire image information of the object and image information of the medical device. For example, the monitoring device 160 may be configured to simultaneously acquire the second image of the object and the key control information of the medical device 110. In some embodiments, the monitoring device 160 may be configured to acquire image information of the object and/or image information of the medical device 110 in real time or at specific intervals.

In some embodiments, the monitoring device 160 may be an image acquisition device. For example, the image acquisition device may be a three-dimensional (3D) camera, such as a TOF (time of flight) camera, a structured light camera, a binocular camera, a lidar camera, or the like, or a combination thereof. The 3D camera may restore the 3D image of the object by determining the position and depth information of the object. In some embodiments, the image acquisition device may be equipped with an infrared camera device. At this time, the obtained image of the object may be an infrared image. The infrared camera device may recognize the posture of the object through a surface temperature of the body of the object. Compared with the optical camera device, the infrared camera device may be weakly affected by a shooting background, shooting light and other factors, thus the accuracy of the infrared camera device may be higher.

In some embodiments, the monitoring device 160 may be configured to acquire video/images in the scanning room. For example, as shown in FIG. 7, the monitoring device 160 may include a first monitoring device 160-1 arranged in the scanning room 101, which may be configured to obtain the video/images of the object entering the scanning room. And the monitoring device 160 may also be configured to obtain the image of the running state of the medical device 110 in the scanning room. In some embodiments, the monitoring device 160 may be configured to acquire video/images in an operation room. For example, as shown in FIG. 10, the monitoring device 160 may include a second monitoring device 160-2 arranged in an operation room 102, which may obtain a key operation image of an operator in the operation room 102, etc. In some embodiments, the monitoring device 160 may include a high-definition camera module of 720P, 1080P, 2K, 4K, etc. In some embodiments, the monitoring device 160 may include any type of camera, including but not limited to still camera, camera, high-speed camera, infrared camera, etc., which may not be limited herein.

It should be noted that the above description of the monitoring system 100 is merely for illustration, and does not limit the scope of application of the present disclosure. For those skilled in the art, various modifications and changes can be made according to the description of the present disclosure. For example, the monitoring system 100 may include a displaying device. For another example, components of the monitoring system 100 may have their own storage device, or may use a common storage device. However, these modifications and changes may not deviate from the scope of the present disclosure.

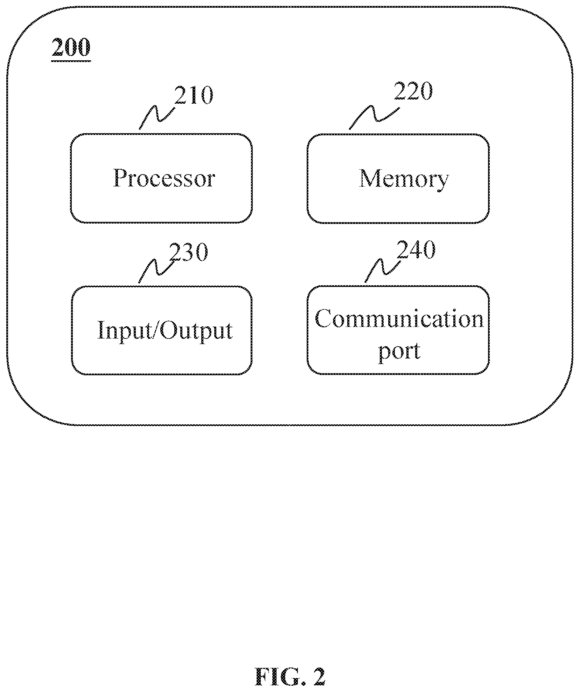

FIG. 2 is a schematic diagram illustrating an exemplary software and/or hardware of a computing device according to some embodiments of the present disclosure.

As shown in FIG. 2, a computing device 200 may include a processor 210, a memory 220, an input/output (I/O) 230, and a communication port 240. In some embodiments, the computing device 200 may be arranged in the processing device 140.

The processor 210 may execute computer instructions (e.g., program codes) and perform functions of the processing device 140 based on techniques described in the present disclosure. The computer instructions may include routines, programs, objects, components, signals, data structures, procedures, modules, and functions that perform specific functions described herein. For example, the processor 210 may obtain motion data of the examination table of the medical device 110 or the scanning data of the medical device 110 from the storage device 150 and/or the terminal 130. In some embodiments, the processor 210 may include a microcontroller, a microprocessor, a reduced instruction set computer (RISC), an application specific integrated circuit (ASIC), an application specific instruction set processor (ASIP), a central processing unit (CPU), a graphics processor (GPU), a physical operation processor (PPU), a microcontroller unit, a digital signal processor (DSP), a field programmable gate array (FPGA), an advanced RISC machine (ARM), a programmable logic device and any circuit or processor capable of performing one or more functions, or any combination thereof.

Merely by way of the example, only one processor is described in the computing device 200. However, it should be noted that the computing device 200 in the present disclosure may include a plurality of processors, so operations of the method performed by one processor described in the present disclosure may be performed by the plurality of processors jointly or separately. For example, if operations A and B are performed by the processor of the computing device 200 in the present disclosure, it should be understood that the operations A and step B may also be performed jointly or separately by two different processors of the computing device 200 (e.g., a first processor performs the operation A, a second processor performs the operation B, or the first processor and second processor jointly perform the operations A and B).

Merely by way of example, the processor 210 may receive instructions following a scanning protocol for imaging/scanning the object. For example, the processor 210 may instruct the examination table 116 of the medical device 110 to move the object into the scanning region 115. For another example, the processor 210 may send a motion termination instruction to the medical device 110 via a controller connected with the medical device 110. As another example, the processor 210 may also provide certain control signals to control a rotation speed and a position of a gantry in the scanning region 115. For another example, the processor 210 may also provide certain control signals to control the on-off of the tube in the scanning region 115. In some embodiments, the processor 210 may acquire scanning data (e.g., the medical image) of the object in the scanning region of the scanning device.

The memory 220 may store data/information obtained from the medical device 110, the terminal 130, the storage device 150, or any other component of the monitoring system 100. In some embodiments, the memory 220 may store one or more programs and/or instructions to perform the methods described in the present disclosure. For example, the memory 220 may store a program for the processor 210 to perform a processing operation, and may also store posture information of the object determined by the processor 210 based on the acquired image data (e.g., the first image). For another example, the memory 220 may store relevant programs for controlling the examination table 116 to perform step motion or continuous motion based on a predetermined trajectory and a predetermined speed. For another example, the memory 220 may store a predetermined correspondence between the operation instruction and the excepted operation information of the medical device.

The input/output (I/O) 230 may input or output signals, data, or information. In some embodiments, the input/output 230 may allow a user to interact with processor 210. In some embodiments, the input/output 230 may include an input device and an output device. The input device may include a keyboard, a mouse, a touch screen, a microphone, a trackball, or the like, or any combination thereof. The output device may include a displaying device, a speaker, a printer, a projector, or the like, or any combination thereof.

Merely by way of example, a user (e.g., an operator) may input data related to an object (e.g., a patient) that is imaged/scanned by the input/output 230. The data related to the object may include identity information (e.g., name, age, gender, medical history, contract information, physical examination results, etc.) and/or include scanning protocol information. The user (i.e., the operator) may also input a parameter required for the operation of the medical device 110, such as an image contrast and/or a ratio, a region of interest (ROI), a slice thickness, an image type, or any combination thereof. The input/output 230 may also display a scanned image generated based on sampling data.

The communication port 240 may be connected with a network (e.g., the network 120) to facilitate data communication. The communication port 240 may establish a connection between the processing device 140 and the medical device 110, the terminal 130, or the storage device 150. The connection may be a wired connection, a wireless connection, or a combination thereof, which may allow transmission and reception of data. In some embodiments, the communication port 240 may be a standardized communication port, such as RS232, RS485, etc. In some embodiments, the communication port 240 may be a specially designed communication port. For example, the communication port 240 may be designed based on the digital imaging and communications in medicine (DICOM) protocol.

It should be noted that the above description of the computing device 200 is merely for illustration, and does not limit the scope of application of the present disclosure. It should be understood that for those skilled in the art, after understanding the principle of the system, any combination of various components may be made, or a subsystem may be formed to connect with other components without departing from this principle. For example, components may share a storage device, or each of the components may have its own storage device. These changes are within the scope of the present disclosure.

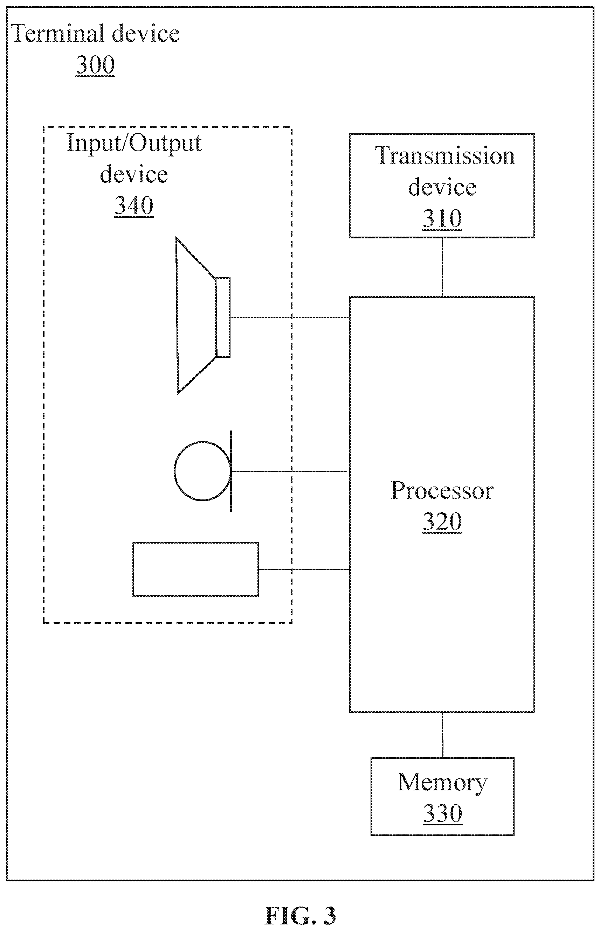

FIG. 3 is a schematic diagram illustrating an exemplary software and/or hardware of a terminal device according to some embodiments of the present disclosure.

As shown in FIG. 3, a terminal device 300 may include one or more (only one is shown in FIG. 3) processors 320 (a processor 320 may include, but be not limited to, a microprocessor, e.g., a microcontroller unit (MCU), or a programmable logic device, e.g., a field programmable gate array (FPGA)), and a memory 330 for storing data. Alternatively, the terminal device 300 may also include a transmission device 310 for communication and an input/output device 340. In some embodiments, the terminal device 300 may be placed in the processing device 140 or the terminal 130. It should be understood by those skilled in the art that structures shown in FIG. 3 is merely for illustration, and does not limit the structures of the above terminal device 300. For example, the terminal device 300 may also include more or fewer components than shown in FIG. 3, or may have a different configuration from that shown in FIG. 3.

The memory 330 may be configured to store control programs, such as software programs and modules of application software, such as the control programs corresponding to method for monitoring a running state of medical device in some embodiments of the present disclosure. The processor 320 may implement the method described in the embodiments of the present disclosure by execute a control program stored in the memory 330 to perform various applications and data processing operations. In some embodiments, the memory 330 may include a high-speed random access memory, a nonvolatile memory, or the like. For example, the nonvolatile memory may include one or more magnetic storage devices, flash memory, or other nonvolatile solid-state memories. In some examples, the memory 330 may further include memory remotely set relative to processor 320, which may be connected with terminal device 300 via a network. Examples of the network described above may include, but be not limited to, the Internet, intranet, local area network (LAN), mobile communication network, or any combination thereof.

The transmission device 310 may be configured to receive or transmit data via a network. Examples of the network described above may include a wireless network provided by a communication provider of the terminal device 300. In one example, the transmission device 310 may include a network interface controller (NIC), which may be connected with other network devices via a base station, to communicate with the Internet. In some embodiments, the transmission device 310 may be a radio frequency (RF) module for communicating with the Internet wirelessly.

FIG. 4 is a block diagram illustrating an exemplary processing device for monitoring the medical device according to some embodiments of the present disclosure. The processing device 400 as described in FIG. 4 may be an exemplary embodiment of the processing device 140 as described in FIG. 2. The processing device 400 may also be referred to as an apparatus for monitoring the medical device (i.e., a monitoring apparatus).

As shown in FIG. 4, in some embodiments, the processing device 400 may include an acquisition module 410, an image analysis module 420, and a control module 430.

The acquisition module 410 may be configured to obtain information such as a plurality of images, or a scanning protocol. In some embodiments, the acquisition module 410 may further include an image acquisition unit 412 and a scanning protocol acquisition unit 414.

In some embodiments, the image acquisition unit 412 may be configured to acquire a plurality of first images in the scanning room by a first monitoring device arranged in the scanning room. In some embodiments, the plurality of first images may include an image of the object and/or a video of the medical device. In some embodiments, when the object is on the examination table, the image acquisition unit 412 may acquire a plurality of second images of the object on the examination table by the first monitoring device. In some embodiments, the image acquisition unit 412 may be configured to acquire a plurality of third images of the object. In some embodiments, the image acquisition unit 412 may be configured to acquire key control information of the medical device as current operation information based on the second monitoring device.

In some embodiments, the scanning protocol acquisition unit 414 may automatically obtain a corresponding scanning protocol based on identity information of the object. In some embodiments, the scanning protocol may include at least predetermined positioning information, a reference rotation speed of a gantry of the medical device, one or more reference parameters associate with anode preheating of a tube of the medical device, and a reference positioning state of each component of the medical device.

The image analysis module 420 may be configured to obtain an analysis result by analyzing the plurality of images. In some embodiments, the image analysis module 420 may be configured to determine mental state information of the object based on the plurality of first images or the plurality of second images of the object, and determine whether the object is suitable for scanning based on the mental state information.

In some embodiments, the image analysis module 420 may further include an identity information determination unit 422, a posture information determination unit 424, a positioning information determination unit 426, and a running information determination unit 428.

In some embodiments, the identity information determination unit 422 may be configured to determine the identity information of the object based on the plurality of first images of the object. In some embodiments, the identity information determination unit 422 may be configured to determine the identity information of the object based on a mark set on the object.

In some embodiments, the posture information determination unit 424 may be configured to determine the posture information of the object based on the plurality of first images of the object.

In some embodiments, the positioning information determination unit 426 may be configured to determine actual positioning information of the object based on the plurality of second images.

In some embodiments, the running information determination unit 428 may be configured to identify actual running information of the medical device based on the video of the medical device. The actual running information may include image information and sound information.

The control module 430 may be configured to control the medical device based on the analysis result. In some embodiments, the control module 430 may further include an examination table control unit 432 and a running control unit 434.

In some embodiments, the examination table control unit 432 may be configured to automatically adjust a height of the examination table of the medical device to a target height based on the posture information of the object. In some embodiments, the examination table control unit 432 may control the medical device to send the object to a target position for scanning when the actual positioning information of the object meet a predetermined condition by comparing the actual positioning information with the predetermined positioning information. In some embodiments, in response to determining that a scanning dose meets a requirement, the examination table control unit 432 may send the object to the target position for scanning. In some embodiments, after the scanning is completed, the examination table control unit 432 may control the examination table to adjust the height of the examination table to the target height. In some embodiments, the examination table control unit 432 may simulate a motion trajectory of the examination table from a position where the object goes to the examination table to the target position, and determine whether an interference involves between the object and the medical device.

In some embodiments, the running control unit 434 may be configured to control the running state of the medical device based on the actual running information and the expected running information. In some embodiments, in response to determining that the medical device is in an abnormal state, the running control unit 434 may send a motion termination instruction to the medical device via the controller connected with the medical device and generate warning information.

In some embodiments, before adjusting the height of the examination table, the control module 430 may adjust a rotation speed of a gantry of the medical device to a rotation speed in the scanning protocol, adjust one or more parameters associated with anode preheating of the tube of the medical device to the one or more reference parameters associate with anode preheating in the scanning protocol, and/or adjust a positioning state of each of the one or more components of the medical device to the reference positioning state in the scanning protocol.

In some embodiments, the control module 430 may generate warning information when an abnormal object exists in the scanning room and/or the object does not exist in the scanning room.

It should be noted that the above description of the processing device 400 is merely for illustration, and is not intended to limit the scope of the present disclosure. For those skilled in the art, various modifications and changes may be made based on the present disclosure. However, these changes and modifications do not depart from the scope of the present disclosure. For example, one or more modules of the above processing device 400 may be omitted or integrated into a single module. As another example, the processing device 400 may include one or more additional modules, such as a storage module for data storage.

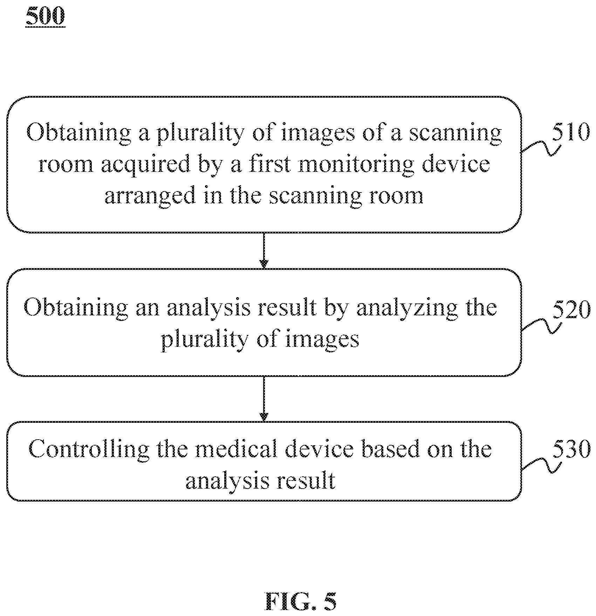

FIG. 5 is a flowchart illustrating an exemplary process for monitoring a medical device according to some embodiments of the present disclosure.

In some embodiments, process 500 may be performed by the monitoring system 100 or the processing device 400. For example, the monitoring method 500 for a medical device may be stored in a storage device (e.g., the storage device 150) in the form of a program or instruction, and the process 500 may be implemented when the monitoring system 100 (e.g., the processing device 140) executes the program or instruction. The operations of 500 of the medical device presented below is illustrative. In some embodiments, process 500 may be implemented with one or more additional operations not described and/or one or more operations not discussed. In addition, a sequence of operations in the process 500 shown in FIG. 5 and described below is not restrictive.

In 510, a plurality of images of a scanning room acquired by a first monitoring device arranged in the scanning room may be obtained. In some embodiments, operation 510 may be performed by the processing device 140, the computing device 200, the terminal device 300, or the processing device 400 (e.g., the acquisition module 410).

The scanning room may refer to a room for accommodating the medical device, such as the scanning room 101 shown in FIG. 7 or FIG. 10. For example, the scanning room may be a room where the medical device (e.g., a CT scanning device, a PET scanning device, the MRI scanning device) is accommodated.

The first monitoring device may include an image acquisition device for obtaining images. In some embodiments, the first monitoring device may include more than one image acquisition device that may be arranged in the scanning room. For example, as shown in FIG. 7, the first monitoring device may include a plurality of image acquisition devices (e.g., 160-1 or 160-2 arranged in the scanning room 101. As also shown in FIG. 10, the first monitoring device may include a plurality of image acquisition devices 160-1 arranged in the scanning room 101.

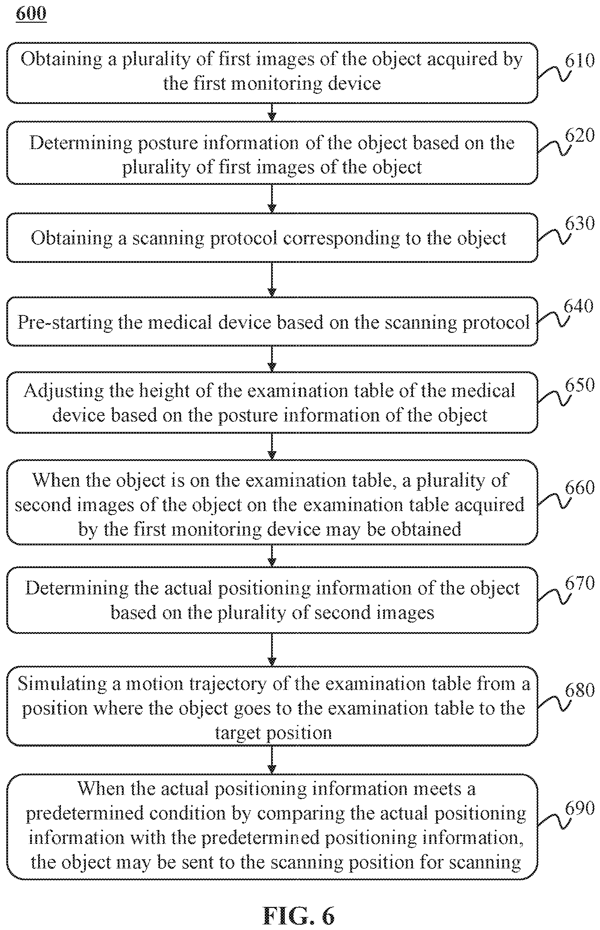

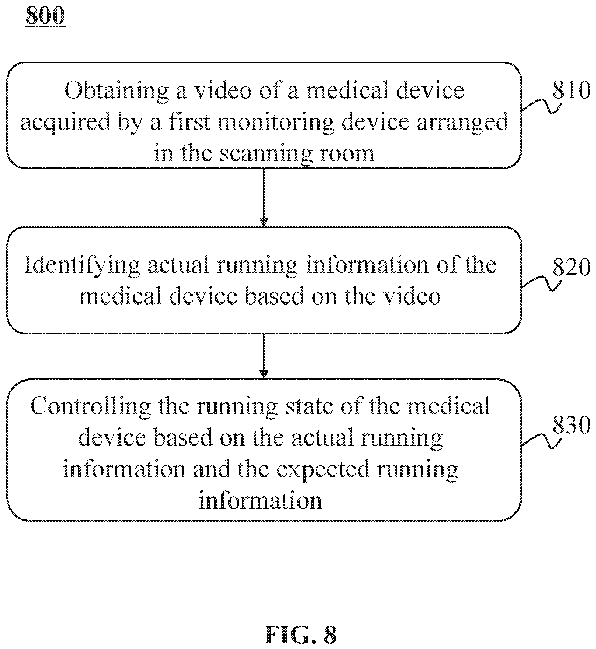

In some embodiments, the plurality of images may include a plurality of images of an object and/or a plurality of images of the medical device. For example, one of the plurality of images may be a first image obtained by the first monitoring device when the object enters the scanning room, or may be a second image obtained by the first monitoring device when the object is on the examination table, or the like. More descriptions of the plurality of images related to the object may be found elsewhere in the present disclosure, for example, FIG. 6 and the descriptions thereof, which may not be described herein. As another example, the plurality of images may be a video of the medical device, or monitoring images of a console obtained by the first monitoring device. More description of the plurality of images of the medical device may be found elsewhere in the present disclosure, for example, FIG. 8 and the descriptions thereof, which may not be described herein.

In 520, an analysis result may be obtained by analyzing the plurality of images. In some embodiments, operation 520 may be performed by the processing device 140, the computing device 200, the terminal device 300, or the processing device 400 (e.g., the image analysis module 420).

In some embodiments, analysis of the plurality of images may include the analysis of an image frame. For example, the processing device 140 may analyze a change of gray, a color distortion of the image frame, or the like. The processing device 140 may extract a feature of the object (e.g., a pupil, a facial makeup, etc.) in the frame image for analysis. In some embodiments, the analysis may include comparison or matching between the plurality of images, or the like.

In some embodiments, the processing device may analyze an image using a trained image recognition model. For example, a trained image recognition model may include a face recognition model, an emotion recognition model, an expression recognition model, an image classification model, or the like, which may be configured to analyze the plurality of images.

In some embodiments, the processing device may determine relevant information of the object and/or relevant information of the medical device by analyzing the plurality of images. For example, posture infonnation and/or identity information of the object may be determined by analyzing a plurality of first images of the object. As another example, actual positioning information of the object may be determined by analyzing a plurality of second images of the object when the object is on the examination table. As still another example, mental state information of the object may be determined by analyzing a plurality of first images or a plurality of second images of the object. As a further example, actual running information of medical device may be determined by analyzing a video of medical device.

In 530, the medical device may be controlled based on the analysis results. In some embodiments, operation 530 may be performed by the processing device 140, the computing device 200, the terminal device 300, or the processing device 400 (e.g., the control module 430).

In some embodiments, the processing device may generate a corresponding control instruction or a control signal based on the analysis result and control the medical device according to the control instruction or the control signal.

In some embodiments, the processing device may control the medical device to adjust a height of an examination table based on the analysis result. For example, the processing device 140 may adjust the height of the examination table 116 of the medical device 110 to a target height based on the posture information of the object. More description of adjusting the height of the examination table based on the posture information may be found elsewhere in the present disclosure, for example, FIG. 6 and the descriptions thereof, which may not be described herein.

In some embodiments, the processing device may control the medical device to send the object to a target position based on the analysis result. For example, the processing device 140 may control the medical device 110 to send the object to the target position (e.g., the scanning region 115) for scanning in response to determining that the actual positioning information meets a predetermined condition by comparing the actual positioning information with predetermined positioning information. More description of the actual positioning information may be found elsewhere in the present disclosure, for example, FIG. 6 and the descriptions thereof, which may not be described herein.

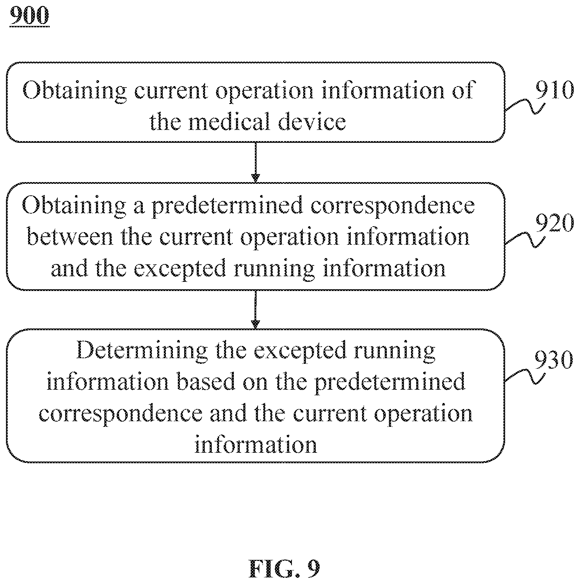

In some embodiments, the processing device may control the running state of the medical device based on the analysis result. For example, the processing device 140 may control the running state of the medical device 110 based on the actual running information and the excepted operation information of the medical device 110. More description of controlling the running state of medical device may be found elsewhere in the present disclosure, for example, FIG. 8 and FIG. 9 and the descriptions thereof, which may not be described herein.

In some embodiments, whether an abnormal object exists in the scanning room may be determined based on a scanning scene and the plurality of images, and warning information may be generated in response to determining that the abnormal object exists in the scanning room.

The abnormal object may be a person unrelated to the scanning, such as a relative of the object, a bystander, a medical staff, or the like. In different scanning scenarios, a person who needs to be in the scanning room may be different. For example, in the scene that the object needs help from the medical staff, the medical staff existing in the scanning room may not be warned, and in other scenes, the medical staff existing in the scanning room may be warned.

In some embodiments, whether an abnormal object exists in the scanning room may be determined based on by determining a count of persons in the image. For example, the count of persons in the image of the scanning room may be identified, and based on the scanning scene, when the count of persons in the image is greater than a count of persons required in the current scanning scene, an abnormal object may exist in the scanning room. In some embodiments, whether an abnormal object exists in the scanning room may be determined by determining identity information of persons in the image. For example, identity information of each person contained in the image may be determined by an image recognition technique, and whether each person is a patient may be determined. When other person who are not patients or the only person in the image is not a patient, the abnormal object existing in the scanning room may be determined.

In some embodiments, the warning information may include voice information, text information, video information, or the like. For example, the voice information reminding the abnormal object to leave may be played via a broadcasting device, the voice information may be "person who is not a patient, please leave as soon as possible!" As another example, the warning information may be displayed on a display screen.

In some embodiments, whether the object is located in a predetermined region may be determined based on the plurality of images, and the warning information may be generated in response to determining that the object is not in the predetermined region.

The predetermined region may refer to a region where the object needs to be located. In some embodiments, the predetermined region may be determined based on different scenes. For example, three stages including before scanning, during scanning, and after scanning may correspond to different regions. For example, the predetermined region before scanning may be a position on the examination table. During scanning, the predetermined region may be the target region of the medical device. After scanning, the predetermined region may be a position next to the examination table. In some embodiments, the predetermined region information may be included in the scanning protocol.

In some embodiments, position information of the object in the scanning room may be recognized based on the first image of the object to determine whether the object is located in a predetermined region. In some embodiments, the position information of the object in the scanning room may be recognized based on the second image of the object to determine whether the object is located in a predetermined region.

In some embodiments, the warning information may include prompt information, movement infonnation, or the like, in response to determining that the object is not in the predetermined region. For example, when the object is not in the predetermined region, a prompt tone may be emitted. As another example, when the object is not in the predetermined region, the object may be reminded to move a specific distance in a specific direction to reach the predetermined region.

In some embodiments, the warning information may be output via broadcasting, displaying, or the like. In some embodiments, when an abnormal object exists in the scanning room or the object is not in the predetermined region, a sound may be used to prompt directly, for example, an alarm tone may be emitted.

It should be noted that the control mode of the processing device to control the medical device may include, but be not limited to one or more control modes described above. It should be noted that the above description of the process 500 is merely for illustration, and does not limit the scope of the present disclosure. For those skilled in the art, various modifications and changes may be made to the process 500 under the guidance of the present disclosure. However, these modifications and changes are still within the scope of the present disclosure.

FIG. 6 is a flowchart illustrating an exemplary process for controlling a medical device according to some embodiments of the present disclosure.

In some embodiments, process 600 may be performed by the monitoring system 100 or the processing device 400. For example, the process 600 may be stored in a storage device (e.g., the storage device 150) in the form of programs or instructions, and the process 600 may be implemented when the monitoring system 100 (e.g., the processing device 140) executes the programs or the instructions. The operations of the process 600 presented below is illustrative. In some embodiments, the process may be implemented with one or more additional operations not described and/or one or more operations not discussed. In addition, an order of the operations of the process 600 shown in FIG. 6 and described below may not intend to be restrictive.

In 610, a plurality of first images of the object acquired by a first monitoring device may be obtained. In some embodiments, operation 610 may be performed by an acquisition module 410 (e.g., the image acquisition unit 412).

In some embodiments, the first monitoring device (e.g., the monitoring device 160 shown in FIG. 1) may be an image acquisition device with a function of data acquisition, storage, and/or transmission. In some embodiments, the image acquisition unit 412 may communicate with the first monitoring device to acquire the plurality of first images of the object.

For example. as shown in FIG. 7, after the object 117 enters the scanning room 101, the first monitoring device (e.g., the image acquisition device 160-1 in FIG. 7) may acquire the plurality of first images of the object 117 entering the scanning room 101. In some embodiments, the plurality of first images acquired by the first monitoring device may be a continuous video of the object entering the scanning room. At this time, the image acquisition unit 412 may divide the continuous video into a plurality of still images, thus the plurality of first images may be acquired. In some embodiments, the plurality of still images (i.e., the plurality of first images) may be acquired based on consistent time intervals. For example, a time interval between any two adjacent images of the plurality still images may be set to 1/24s (i.e., obtaining 24 frames of images with an equal time interval within 1 second). In some embodiments, the time interval between any two adjacent images of the plurality of still images may be specifically set based on actual requirements. For example, the time interval may be specifically set as 1/25s, 1/48S, or 1/60swhich means that 25, 48, and 60 frames of images may be obtained with an equal time intervals within 1 second, respectively. Setting the time interval for obtaining the plurality of still images may exclude some images with poor image quality (e.g., an overexposed image, or an image with afterimage), thus the plurality of still images may maintain a high image quality, thus making the posture information extracted based on the images in subsequent operations more accurate.

In 620, posture information of the object may be determined based on the plurality of first images of the object. In some embodiments, operation 620 may be performed by the image analysis module 420 (e.g., the posture information determination unit 424).

In some embodiments, the posture information determination unit 424 may perform operations such as gray-scale change, and/or color distortion on the plurality of first images of the object entering the scanning room acquired in operation 610, obtain contours of the object represented in the plurality of first images, and determine the posture information of the object based on a comparison between the contours and a predetermined contour.

In some embodiments, the posture information determination unit 424 may determine feature points of the object based on the plurality of first images, and determine the posture information based on the feature points. The posture information may include a sitting posture, a standing posture, a lying posture (e.g., a supine posture, a lateral posture), or other postures. For example, coordinates of the feature points may be extracted from the plurality of first images using an algorithm, for example, OpenPose, Hourglass Network, Mask RCNN, or the like. Taking leg joints of the object as an example, the coordinates of each of the leg joints of the object (e.g., a knee joint, an ankle joint, a cross joint, etc.) in each first image may be obtained based on at least one of the above algorithms. As another example, feature point information of the body of the object may be obtained based on at least one of above algorithms.

In some embodiments, the posture information determination unit 424 may determine whether the object is in a lying posture based on the feature point information of the body of the object. For example, when feature points of the body of the object are horizontally distributed or close to horizontally distributed, the object may be determined that the object is lying on the examination table.

In some embodiments, when determining that the body of the object is in an upright state, the posture information determination unit 424 may determine whether the object is in a sitting posture (e.g., sitting in a wheelchair) or a standing posture based on the leg joints of the object. Specifically, the posture information determination unit 424 may connect the knee joint with the ankle joint by a line segment, and connect the knee joint with the cross joint by another line segment based on the coordinates of each joint of the leg joints, thus the posture information of the object may be determined based on an angle between the two line segments (the angle between the two line segments may be within 0° ~90°). For example, the line segment between the knee joint and the ankle joint may be line segment A, and the line segment between the knee joint and the cross joint may be line segment B. When an angle between the line segment A and the line segment B is greater than a predetermined threshold (e.g., the predetermined threshold is 45 °), the object may be considered to be in a sitting posture. As another example, when the angle between the line segment A and the line segment B is relatively small (e.g., close to 0°), the object may be considered to be in a standing posture.

Because the plurality of still images may be interfered by a light source, a shooting background, lighting, or other factors, the coordinates of the feature points may have some noises. For example, the noises of the plurality of still images may include Gaussian noise, non-Gaussian noise, systematic noise, or the like, or any combination thereof. In some embodiments, the coordinates of the feature points may be de-noised to obtain de-noised coordinates of the feature points. For example, Kalman filtering may be used to filter the noise of a still image. Kalman filter is an algorithm that estimating a system state based on input and output observation data of the system by using a system state equation. In some embodiments, Kalman filtering may include a linear Kalman filtering, an extended Kalman filtering, a progressive extended Kalman filtering, an unscented Kalman filtering, or other filtering methods, or any combination thereof.

In 630, a scanning protocol corresponding to the object may be obtained. In some embodiments, the operation 630 may be performed by the acquisition module 410 (e.g., the scanning protocol acquisition unit 414).

The scanning protocol may include information related to reference scanning parameters associated with scanning and/or reference reconstruction parameters associated with the scanning. For example, taking the CT scanning device as an example, the reference scanning parameters may include a voltage (kV) of the tube, a current (mA) of the tube, a total exposure time, a scanning type (e.g., spiral scanning, axial scanning), a field of view (FOV), a pitch, a tilt angle of the gantry, a rotation time of the gantry, a slice thickness, or the like. The reference image reconstruction parameters may include a reconstruction FOV, a reconstruction slice thickness, a reconstruction interval for image reconstruction, a window width (WW), a window level (WL), a reconstruction matrix, a reconstruction algorithm (e.g., a filtered back projection algorithm, a fanbeam reconstruction algorithm, an iterative reconstruction algorithm, etc.).

In some embodiments, the scanning protocol may include predetermined positioning information. The positioning information may reflect a region where the object is scanned and photographed. In some embodiments, the scanning protocol may include information such as a reference rotation speed of a gantry of the medical device, one or more reference parameters associated with anode preheating of a tube of the medical device, and a reference positioning state (e.g., a tilt angle of the gantry, a pitch, a FOV, etc.) of each component of the medical device.

In some embodiments, the scanning protocol acquisition unit 414 may obtain the scanning protocol corresponding to the object based on the identity information of the object. In some embodiments, the scanning protocol may be provided by an operator (e.g., a doctor or a technician).

In some embodiments, the identity information of the object may be determined based on the plurality of first images of the object. In some embodiments, the identity information of the object may be determined by a mark set on the object.

In some embodiments, the identity information determination unit 422 may obtain a face image of the object based on the plurality of first images of the object, and then determine the identity information of the object based on the face image.

In some embodiments, the object may be provided with a mark. The mark may be set as a two-dimensional code, a wrist information code, radio frequency identification (RFID), an integrated circuit (IC) card, or the like. For example, the object may present the mark (e.g., the wrist information code) when entering the scanning room, and the identity information determination unit 422 may acquire the identity information of the object based on the mark.

In some embodiments, the mark may be set as a biometric feature, including a fingerprint, a palmprint, a pupil, a pupillary distance, a voiceprint, a facial makeup, a phalanx, the skull, or the like, or any combination thereof. These biometric features may be marked in advance and may be stored in a database. After acquiring a biometric feature of the object (e.g., a pupil of the object), the identity information determination unit 422 may match the biometric feature with the biometric features stored in the database to obtain the identity information of the object.

In 640, the medical device may be pre-started based on the scanning protocol. In some embodiments, operation 640 may be performed by the control module 430.

In some embodiments, the control module 430 may pre-start the medical device based on the scanning protocol. In some embodiments, the control module 430 may adjust the rotation speed of the gantry of the medical device (e.g., the medical device 110) according to the reference rotation speed in the scanning protocol based on the scanning protocol. For example, the control module 430 may adjust the rotation speed of the gantry of the medical device (e.g., the medical device 110) to be equal to the reference rotation speed in the scanning protocol. In some embodiments, the control module 430 may adjust one or more parameters associated with anode preheating of the tube of the medical device (e.g., the medical device 110) according to the one or more reference parameters associated with anode preheating in the scanning protocol based on the scanning protocol. For example, the control module 430 may adjust the voltage and the current of the tube of the medical device 110 to a reference value corresponding to the voltage of the tube and the current of the tube in the scanning protocol. In some embodiments, the control module 430 may adjust the positioning state of each component of the scanning device according to the reference positioning state in the scanning protocol based on the scanning protocol. For example, the control module 430 may rotate the medical device 110 to a corresponding rack tilt angle, a pitch, a FOV, or the like.

In 650, the height of the examination table of the medical device may be adjusted based on the posture information of the object. In some embodiments, operation 650 may be performed by the control module 430 (e.g., the examination table control unit 432).

In some embodiments, the examination table control unit 432 may determine the target height of the examination table when the object goes to the examination table based on the posture information of the object. It can be understood that an appropriate height of the examination table may facilitate a motion of the object to the examination table.

In some embodiments, when the object is detected to walk into the scanning room, the examination table control unit 432 may calculate a length of a leg of the object based on the posture information of the object, and lower the height of the examination table when the object goes to the examination table to below the length of the leg of the object (e.g., the height of the examination table may be 100 mm below the length of the leg). For example, when the posture information is extracted based on the feature points, the examination table control unit 432 may acquire a height of the cross joint of the object, and further adjust the height of the examination table to below the height of the cross joint to facilitate the object to go to the examination table.

In some embodiments, when the object is detected to enter the scanning room in a wheelchair, the examination table control unit 432 may acquire a height of the wheelchair and lower the height of the examination table to below the height of the wheelchair. In some embodiments, the height of the wheelchair may be a standard height (e.g., a uniform height of all wheelchairs in a hospital). When the posture information obtained in the operation 620 is that the object enters the scanning room in the wheelchair, the examination table control unit 432 may directly lower the height of the examination table to below the standard height of the wheelchair. In some embodiments, the examination table control unit 432 may determine the height of the wheelchair based on the posture information of the object. Specifically, the examination table control unit 432 may directly acquire a height of a joint of the object (e.g., a height of the knee joint of the object), and determine the height of the knee joint as the height of the wheelchair, and further adjust the height of the examination table to be below the height of the knee joint of the object.

In some embodiments, when the object is detected to enter the scanning room on a stretcher, the examination table control unit 432 may acquire a height of the stretcher and lower the height of the examination table to below the height of the stretcher. In some embodiments, the height of the stretcher may be a standard height (e.g., a uniform height of all stretchers in the hospital). When the posture information obtained in the operation 620 is that the object enters the scanning room on the stretcher, the examination table control unit 432 may directly lower the height of the examination table to below the standard height of the stretcher. In some embodiments, the examination table control unit 432 may determine the height of the stretcher based on the posture information of the object. In some embodiments, the examination table control unit 432 may directly acquire the height (e.g., an average value of the leg, a trunk and a head of the object) of the joint of the object, determine the average value of the height of each joint as the stretcher height, and further adjust the height of the examination table to below the height of the stretcher of the object.

In some embodiments, after the examination table is adjusted to an appropriate height (e.g., the target height), prompt information may be sent to the object to prompt the object to go to the examination table. In some embodiments, a way to prompt the object may include broadcasting, sound prompt, image prompt, text prompt, video prompt, or the like. For example, the prompt information may be output to the object via the terminal 130 with a voice or a text of "Mr./Ms. XX, the height of the examination table has been adjusted already for you, please go to the examination table for a scan." As another example, a sound output device in the scanning room may emit a sound such as "beep" to prompt the object to go to the examination table.

In some embodiments, the prompt information may include a motion distance and/or a motion direction. For example, the prompt information may be: "please move forward two meters before going to the examination table."

It should be noted that the way to prompt may include others, such as alternating signal lights of different colors (e.g., red, green, etc.) to prompt the object, which may not be limited in the present disclosure.

After the examination table is lowered to the target height, and the object goes to the examination table (e.g., lying on the examination table 116), the subsequent operation may be performed.

In 660, when the object is on the examination table, a plurality of second images of the object on the examination table acquired by the first monitoring device may be obtained. In some embodiments, operation 660 may be performed by the acquisition module 410 (e.g., the image acquisition unit 412).

When the object is on the examination table, the image acquisition unit 412 may acquire the plurality of second images of the object on the examination table. In some embodiments, the image acquisition unit 412 may communicate with an image acquisition device. After a posture on the examination table of the object is determined, the image acquisition unit 412 may acquire the plurality of second images of the object by the image acquisition device. In some embodiments, the plurality of second images may be acquired by dividing a continuous video (e.g., obtaining a plurality of still images frame by frame). More descriptions of dividing the continuous video to obtain the plurality of still images may be found elsewhere in the present disclosure, for example, operation 620, which may not be described herein.

In 670, actual positioning information of the object may be determined based on the plurality of second images. In some embodiments, t operation 670 may be performed by the image analysis module 420 (e.g., the positioning information determination unit 426).

In some embodiments, the positioning information determination unit 426 may determine a scanning posture of the object and a positional relationship between the object and the examination table based on the plurality of second images of the object, and further determine the actual positioning information of the object based on the scanning posture and the positional relationship. In some embodiments, the positioning information determination unit 426 may perform one or more operations such as gray-scale change and/or color distortion on the plurality of second images of the object to obtain the contours of the object represented in the plurality of second images, and then determine the scanning posture of the object and the positional relationship between the object and the examination table based on a comparison of the contours. In some embodiments, the scanning posture of the object may include a supine posture, a lateral posture, a hand raising, or the like. In some embodiments, the positional relationship between the object and the examination table may be measured by a coordinate system. For example, the coordinate system may be established based on the examination table, and the coordinate system may be calibrated for each feature point or contour of the object to obtain corresponding coordinates.

In some embodiments, the positioning information determination unit 426 may acquire the scanning posture of the object and the positional relationship between the object and the examination table by acquiring 3D contour data of the object. The 3D contour data may be data reflecting a body shape contour of the object. In some embodiments, the positioning information determination unit 426 may accurately detect a distance between a point in an image of the object and the camera by a 3D imaging device, thereby acquiring 3D spatial coordinates of the point in a second image of the object, and then obtain the 3D contour data (i.e., a 3D model) of the object by modeling based on the three-dimensional spatial coordinates. In some embodiments, the positioning information determination unit 426 may acquire two-dimensional (2D) image data of the object by several 2D imaging devices, and then perform 3D reconstruction based on the 2D image data to obtain the 3D contour data of the object.

It should be understood that the 3D contour data of the object acquired by the image acquisition device may include a relative distance and a relative angle between a point in the 3D contour data of the object and the 3D imaging device, or a relative distance and a relative angle between a point in the 3D contour data of the object and the 2D imaging device. Since a position (a distance and an angle) of the 3D imaging device or the 2D imaging device relative to the examination table is determined, a relative position of the object and the examination table may be obtained by processing the above data.

In some embodiments, the positioning information determination unit 426 may further determine the feature points of the object based on the plurality of second images, and further determine the scanning posture of the object and the positional relationship between the object and the examination table. For example, the coordinates of feature points may be extracted from the plurality of second images by using an algorithm, such as OpenPose, an Hourglass Network, a Rask RCNN, or the like. The more descriptions of acquiring the scanning posture based on the feature points may be found elsewhere in the present disclosure, for example, the operation 620, and may not be described herein.

In some embodiments, the positioning information determination unit 426 may determine the actual positioning information based on the scanning posture of the object and the positional relationship between the object and the examination table. The actual positioning information may reflect an actual scanning region of the object under a condition of the current scanning posture of the object and the positional relationship between the object and the examination table. It should be understood that the positioning information determination unit 426 may determine a current photographed position (i.e., the actual scanning region) based on the scanning posture of the object and the positional relationship between the object and the examination table.

In some embodiments, the positioning information determination unit 426 may compare the actual positioning information with predetermined positioning information (or reference positioning information) in the scanning protocol to obtain a matching degree. It should be understood that the predetermined positioning information in the scanning protocol may reflect a target region (e.g., a part in the body of the object corresponding to a nidus) where the object needs to be scanned. For example, when a chest X-ray scan is performed on the object, the chest is a photographed position. In some embodiments, the predetermined positioning information in the scanning protocol may be a part in the body that has been determined by a doctor before photographing. And the actual positioning information may reflect the part in the body to be photographed with the position of the object and the scanning posture. In some embodiments, the actual positioning information may deviate from the predetermined positioning information because a position of the object may be shifted when the object lying on the examination table. For example, a target position corresponding to the predetermined positioning information may be the chest, but a target position corresponding to the actual positioning information may be the abdomen.

In some embodiments, the positioning information determination unit 426 may compare the actual positioning information with the predetermined positioning information in the scanning protocol to obtain the matching degree. The matching degree may be represented in a variety of ways, for example, a similarity of positioning information. The matching degree may reflect the similarity between the actual positioning information and the predetermined positioning information in the scanning protocol. For example, the matching degree may be measured by a deviation value of coordinates between two kinds of positioning information and a statistical deviation value of the deviation value of the coordinates (e.g., an average deviation of the coordinates). As another example, the matching degree may reflect a level of the similarity. For example, the level of the similarity may include three levels: 1, 2 and 3. The higher the level of the similarity is, the greater a degree of similarity is As another example, the matching degree may reflect a size of the same region between the actual positioning information and the predetermined positioning information in the scanning protocol. For example, a similarity between two regions corresponding to the actual positioning information and the predetermined positioning information respectively may be 80%, 70%, 35%, or the like. It should be understood that when a degree of similarity between the two regions is 80%, it may represent that a percentage of 80% of the two regions between the actual positioning information and the predetermined positioning information in the scanning protocol are similar.

In some embodiments, the positioning information determination unit 426 may compare the actual positioning information with the predetermined positioning information in the scanning protocol by using a machine learning model. For example, the machine learning model may be a convolutional neural network. In the convolutional neural network, the actual positioning information and the predetermined positioning information in the scanning protocol input into the convolutional neural network may be represented by picture matrixes, and an output of the convolutional neural network may be a similarity between the actual positioning infonnation and the predetermined positioning information. For example, a row of the picture matrix may represent a length of a region corresponding to positioning information (i.e., the actual positioning information or the predetermined positioning information), a column of the picture matrix may represent a width of the region, and elements of the picture matrix may correspond to pixels (color degrees) of the region. In some embodiments, the input of the convolutional neural network may be the picture matrixes corresponding to the actual positioning information and the predetermined positioning information in the scanning protocol, and the output of the convolutional neural network may be the similarity between the actual positioning information and the predetermined positioning information. A mapping relationship between the actual positioning information and the predetermined positioning information in the scanning protocol may be constructed by the convolutional neural network, and more accurate comparison results may be obtained.

In some embodiments, when the actual positioning information does not meet the predetermined condition by comparing the actual positioning information with the predetermined positioning information, the actual positioning of the object on the examination table, that is, the scanning posture of the object and/or the positional relationship between the object and the examination table, may be adjusted to meet the predetermined condition.

In some embodiments, when the actual positioning information does not meet the predetermined condition by comparing the actual positioning information with the predetermined positioning information, the object may be prompted. For example, the prompt information may include voice prompt information and/or a correct positioning image. In some embodiments, a prompt content of the prompt information may include adjusting a direction, an angle, a distance, or the like. For example, in a scene where the chest of the object needs to be scanned, when a right arm of the object is in front of the chest, the monitoring system 100 may generate a prompt message "Mr./Mrs. Li, please move your right hand away from the chest, and put the right hand on right side of your body!" In some embodiments, the monitoring system 100 may display the correct positioning image to the object via an output device, so that the object may adjust position based on the correct positioning image.

In 690, when the actual positioning information meets a predetermined condition by comparing the actual positioning information with the predetermined positioning information, the object may be sent to the scanning position for scanning. In some embodiments, operation 690 may be performed by the control module 430 (e.g., the examination table control unit 432).

In some embodiments, a scanning dose of the object may be predetermined based on the scanning protocol. For example, the scanning dose may be stored in the scanning protocol in advance, which may be called directly when the medical device performs a scan on the object.

In some embodiments, the scanning dose may be determined in real time based on the scanning posture of the object and the actual scanning region, that is, based on the actual positioning information of the object. In the embodiment, the relative position of a part to be photographed and the gantry corresponding to the actual positioning information, a thickness, width and height of the part to be photographed may be obtained, thus an appropriate scanning dose may be determined.

In some embodiments, the scanning dose corresponding to different positioning information may be determined based on statistical data, that is, a correspondence between the positioning information and the scanning dose. The statistical data may be historical diagnosis data from various sources, for example, a scanning image, positioning information, a scanning dose, a target position, patient information, or other data during diagnosis in different regions, different hospitals or different patients.

In some embodiments, the historical diagnostic data may be analyzed and processed by modeling or using an analysis algorithm with various data, such as statistical analysis, discriminant analysis, etc., to determine scanning doses corresponding to different positioning information. For example, scanning doses used by a plurality of patients of the same age (e.g., 50-55 years old), the same gender, or the same scanning region during a diagnosis may be counted, thus a scanning dose used by a largest count of patients whose condition is the same with the object may be designated as the scanning dose when a scan is performed on the object.

In some embodiments, the appropriate scanning dose may be adjusted based on the actual positioning information of the object based on the correspondence between different positioning information and the scanning dose.

In some embodiments, a trained scanning dose determination model may be used to determine the scanning dose of the object based on the actual positioning information of the object.

The scanning dose may include an absolute value, a value within a range, etc. For example, different positioning information may correspond to a minimum and/or maximum of different scanning doses respectively.

In some embodiments, the scanning dose of the object may be determined based on case information and/or basic information of the object. The case information may be historical diagnosis information of the patient. For example, based on the actual positioning information and/or the basic information of the object, a case record may be queried and matched in the storage device 150 based on a query instruction, and a corresponding scanning dose of the matched case record may be designated as the scanning dose of the object.

In some embodiments, the determined scanning dose may be store in the scanning protocol for a confimation by an operator (e.g., a doctor, or a nurse). In some embodiments, after confirming that the scanning dose meets a requirement, the examination table control unit 432 may control the examination table to send the object to the target position for scanning.

In some embodiments, after the object is sent to the target position, the running control unit 434 may light an exposure button and wait for the operator to press the exposure button. In some embodiments, the running control unit 434 may scan the object based on an exposure instruction (e.g., an exposure button is pressed by the operator).

In some embodiments, in the process of scanning the object, a running state of the medical device (e.g., the medical device 110) may be monitored in real time by the monitoring device (e.g., the first monitoring device 160-1 and the second monitoring device 160-2). When the medical device is in an abnormal state, the medical device may be stopped to run and/or warning information may be generated based on the abnormal state. For example, when the monitoring device 160 recognizes that the medical device moves without a key being touched, the key is not rebounded after a staff pressing the key, or the actual running information of the medical device is inconsistent with the standard operation process, a termination instruction may be sent to the medical device and the warning information may be generated. The more description of monitoring the medical device may be found elsewhere in the present disclosure, for example, FIG. 8, and FIG. 9 and the descriptions thereof, which may not be described herein.

In some embodiments, historical scanning protocols may be searched based on a scanning region corresponding to the actual positioning information of the object to determine whether there is a matched scanning protocol. If there is a scanning protocol in the history scanning protocols that the part to be photographed in the scanning protocol is the same with a part to be photographed corresponding to the actual positioning information and a difference between predetermined posture information in the scanning protocol and actual posture information is within a range of a predetermined threshold, a scanning protocol matching the actual positioning information may be determined in the history scanning protocols. Otherwise, there may be no scanning protocol matching the actual positioning information in the historical scanning protocols.

In some embodiments, after determining a scanning region (e.g., the part to be photographed and size data of the part to be photographed) corresponding to the actual positioning information of the object, one or more historical scanning protocols may be searched based on a searching keyword (e.g., a chest or a chest X-ray), and several historical scanning protocols may be selected as candidate scanning protocols. A candidate scanning protocol may be a historical scanning protocol that matches the searching keyword. In some embodiments, the posture information of a historical object in the candidate scanning protocol may be compared with the posture information of the object, and the candidate scanning protocol whose comparison result is within a range of a threshold may be regarded as the scanning protocol. In some embodiments, the searching keyword may be one or more parameters included in the scanning protocol. For example, the searching keyword may be the positioning information (e.g., standing photographing, lateral photographing, supine photographing, etc.) of the object in the scanning protocol, and the part to be photographed (e.g., the head, the chest, etc.).

In some embodiments, the scanning region corresponding to the actual positioning information may be compared with a region corresponding to the historical scanning protocol from many aspects. For example, a height of the object may be compared a height of a historical object in the historical scanning protocol. As another example, a thickness, a width and a height of the part to be photographed of the object may be compared with thickness, a width and a height of the part to be photographed of the historical object. When a comparison result is greater than a threshold, there may be no matching candidate scanning protocol. For example, the thickness of the part to be photographed (e.g., a chest) of the object is 30cm, while the thickness of the part to be photographed (e.g., a chest) in the candidate scanning protocol is 40cm or 20cm. A difference between the thickness of the part to be photographed of the object and the thickness of the part to be photographed in the candidate scanning protocol is greater than a threshold (e.g., 3cm, 5cm, 7cm, etc.), the candidate scanning protocol may be considered as not matching the actual positioning information. When the comparison result is smaller than or equal to the threshold, there may be a matching candidate scanning protocol. For example, the thickness of the part to be photographed (e.g., a chest) of the object is 30cm, and the thickness of the part to be photographed (e.g., a chest) in the candidate scanning protocol is 35cm or 25cm. A difference between the thickness of the part to be photographed of the object and the thickness of the part to be photographed in the candidate scanning protocol is within a range of the threshold (e.g., 5cm, 7cm, etc.), thus the candidate scanning protocol may be considered as matching the actual positioning information.

In some embodiments, after the scanning is completed (e.g., after the operator presses a completion button or after scanning data has been obtained by the medical device), the examination table control unit 432 may control the examination table to retuni to a position where the object goes to the examination, thus the object may get out of the examination table. In some embodiments, after the scanning is completed, the examination table control unit 432 may control the examination table to adjust to the height of the examination table to the target height.

In some embodiments, before performing the operation 690, the process 600 may further include the operation 680. In 680, a motion trajectory of the examination table from a position where the object goes to the examination table to the target position may be simulated. In some embodiments, operation 680 may be performed by the control module 430 (e.g., the examination table control unit 432).

In some embodiments, the control module 430 may simulate the motion trajectory of the examination table from a position where the object goes to the examination table to the target position based on a predetermined target position, a position where the object goes to the examination table, and a correspondence between the object and the examination table. For example, an interference region corresponding to the examination table may be determined based on the examination table and the motion trajectory from a position where the object goes to the examination table to the target position, and whether an interference involves between the object and one or more components (e.g., the gantry, etc.) of the medical device may be determined based on the interference region. If the interference does not involve between the object and one or more components, operation 690 may be performed; otherwise, a scanning posture of the object may need to be adjusted to eliminate a risk of the interference.

In some embodiments, the motion trajectory may be planned based on a simulation result, and the examination table may be controlled to move to the target position based on the planned motion trajectory. For example, the planned motion trajectory may include control information such as a motion direction, a motion distance, swing or not and an angle of the examination table. The planned motion trajectory may avoid the interference region to avoid the interference

In some embodiments, after the scanning is completed, the motion trajectory of the examination table may be planned again to control a return of the examination table (e.g., the examination table may return from the target position to the position where the object goes to the examination table).

In some embodiments, before scanning, whether the object is suitable for scanning may be determined. In some embodiments, mental state information of the object may be determined based on a plurality of third images of the object, and whether the object is suitable for scanning may be determined based on the mental state information. For example, the plurality of third images of the object may be input into a trained mental state recognition model to recognize the mental state information of the object. The mental state information may include rosy cheeks, pathological cheeks, or the like.

In some embodiments, the plurality of third images may include an image when the object enters the scanning room (e.g., a first image), an image before the object goes to the examination table, an image when the object is on the examination table (e.g., a second image), or an image when the object is in the target position. In some embodiments, the plurality of third images may be a continuous video of the object during the process of scanning. In some embodiments, the plurality of third images of the object may be acquired by the first monitoring device.

When the object is in a poor mental state and not suitable for scanning, the scan may be canceled or interrupted. In some embodiments, the mental state of the object may be monitored in real time during the process of scanning, and when the mental state of the object is abnormal, the scanning may be terminated in time.

In the embodiments of the present disclosure, the posture information of the object may be acquired by the monitoring device, and the examination table may be automatically lifted and lowered. And the medical device may be pre-started by calling the scanning protocol based on the identity information, thus the convenience of scanning may be improved, time of the process of scanning may be reduced, and operations of the process of scanning may be performed rapidly.

It should be noted that the above description of the process 600 is merely for illustration, and does not limit the scope of the present disclosure. For those skilled in the art, various modifications and changes may be made to the process 600 under the guidance of the present disclosure. However, these modifications and changes are still within the scope of the present disclosure.

FIG. 7 is a schematic diagram illustrating an exemplary arrangement of a medical device according to some embodiments of the present disclosure.

FIG. 7 shows a scanning room 101 (a ceiling and a side of wall are not shown in order to show an internal structure of the scanning room), and the medical device 110 (including the examination table 116 and the scanning region 115) may be arranged in the scanning room 101. More descriptions of the medical device 110 may be found elsewhere in the present disclosure, for example, FIG. 1 and the descriptions thereof, which may not be described herein.

In some embodiments, the monitoring device 160 (e.g., 160-1 or 160-2 shown in FIG. 7) may be arranged on a wall. In some embodiments, the monitoring device 160 may be arranged on the ceiling of the scanning room 101. The monitoring device 160 may be arranged on the ceiling to obtain a broader view, thus avoiding the view being blocked by other institutions or objects.

In some embodiments, the monitoring device 160 may include only one camera. In this case, the camera may be arranged above the examination table 116. In some embodiments, the monitoring device 160 may include at least two (e.g., two, three, four, five, or six, etc.) cameras. By setting multiple cameras, the image acquisition field of view of the monitoring device 160 may be effectively increased to avoid the inaccurate image acquisition caused by the blocked area of the field of view of one camera. In some embodiments, two or more cameras may be configured to obtain a 3D image of the object. For example, the two or more cameras may transmit obtained images to the processing device (e.g., the processing device 140), and the processing device may convert 2D images into the 3D images of the object based on the images obtained by the two or more camera with a way of image mosaic, image coordinate conversion, or the like.

It should be noted that those skilled in the art may make various reasonable changes to the technical scheme of the present disclosure based on the basis of the present disclosure. For example, a count of cameras in the monitoring device 160, the arrangement of cameras, and/or positions of cameras may be determined based on an actual requirement. For example, the arrangement of cameras may include, but be not limited to, array arrangement, ring arrangement, topology arrangement, or the like. Such changes are still within the scope of the present disclosure.

In some embodiments, in the process of using medical device (e.g., a single-mode scanning device such as a PET scanning device, a CT scanning device. an MRI scanning device, and/or a multi-mode scanning device such as a PET-CT, PET-MRI), an abnormal condition such as keys of the medical device being stuck, or abnormal motion of the medical device may occur when there is no medical staff. In related technologies, a sensor (e.g., a force sensor, an infrared sensor, etc.) may be installed on a motion component of the medical device to detect abnormal motion. However, in this case, only when a degree of the abnormal motion of the motion component reaches a certain threshold, the abnormal motion may be detected. Thus, the detection accuracy may be low. Some embodiments of the present disclosure may provide a method for monitoring the running state of medical device (for example, process 800 and process 900), which may monitor the running state of the medical device, thus the abnormal motion of the medical device may be detected in real-time.

FIG. 8 is a flowchart illustrating an exemplary process for monitoring a running state of a medical device according to some embodiments of the present disclosure.

In some embodiments, the process 800 for monitoring the running state of the medical device may be performed by the monitoring system 100 or the processing device 400. For example, the process 800 may be stored in a storage device (e.g., storage device 150) in the form of programs or instructions, and process 800 may be implemented when the monitoring system 100 (e.g., processing device 140) executes the programs or instructions. The operations of the process 800 presented below is illustrative. In some embodiments, the process of the process 800 may be implemented with one or more additional operations not described and/or one or more operations not discussed. In addition, the order of operations of the process 800 shown in FIG. 8 and described below may not intend to be restrictive.

In 810, a video of a medical device acquired by a first monitoring device arranged in the scanning room may be obtained. In some embodiments, operation 810 may be performed by the acquisition module 410 (e.g., the image acquisition unit 412).

In some embodiments, the first monitoring device (e.g., a camera) may be arranged in the scanning room to monitor the running of the medical device in real time with video and obtain the video of the medical device.

In some embodiments, the video may be a monitoring video of the medical device obtained in real time by the first monitoring device. In some embodiments, the video may include a running performance of the examination table and a gantry of the medical device.

In some embodiments, when the object enters the scanning room, the video of the medical device may be obtained by the first monitoring device arranged in the scanning room. In some embodiments, when the object is on the examination table, the video of the medical device may be obtained by the first monitoring device arranged in the scanning room. In some embodiments, in response to determining that actual positioning information meets a predetermined condition based on a plurality of second images of the object, the video of the medical device may be obtained by the first monitoring device set in the scanning room. In some embodiments, when controlling the medical device to send the object to the target position, the video of the medical device may be obtained by the first monitoring device arranged in the scanning room.

In 820, actual running information of the medical device may be identified based on the video. In some embodiments, operation 820 may be performed by the image analysis module 420 (e.g., the running information determination unit 428).

The actual running information may be actual feedback of the medical device on the current operation information. The actual running information and the current operation information may be obtained simultaneously or asynchronously. For example, if there is no time interval between the current operation information and corresponding feedback, the actual running information may be obtained at the same time as the current operation information. If there is a time interval between the current operation information and the feedback, there is also a time interval between a time of obtaining the actual running information and a time of obtaining the current operation information.

The current operation information may refer to an input instruction information currently obtained by the medical device, which may be configured to control an operation of the medical device. For example, the current operation information may include pressing a key of "scanning", selecting "scanning protocol A", inputting a scanning parameter, or the like. In some embodiments, the current operation information may include internal control information and/or key control information.

In some embodiments, the internal control information of the medical device may be acquired by a controller connected with the medical device.

In some embodiments, the monitoring system (e.g., the monitoring system 100) may include a medical device and a console. The console may include a controller and keys as shown in FIG. 10. The controller may be configured to send the internal control information to the medical device, and may receive a scanning result returned by the medical device. The internal control information may be information sent by the controller to the medical device and configured to control the running of the medical device.

In some embodiments of the present disclosure, the current operation information may be obtained based on the internal control information and/or key control infonnation in the controller, thus the adaptability of the method in different scenarios may be improved.

In some embodiments, the key control information of the medical device may be acquired by a second monitoring device. In some embodiments, the key control information may include state information such as pressing or rebounding of a key. Keys may include user interfaces allowing a user to operate the medical device.

As shown in FIG. 10, the second monitoring device 160-2 may be arranged in the operation room 102 that controls the medical device, or may be arranged in the scanning room 101 where the medical device is located. In the operation room, the medical staff may input the internal control information to the medical device via a key on the console. When inputting different internal control information, states of different keys on the console may be different. By the second monitoring device, a key monitoring image reflecting a state of pressing or rebounding of the key on the console may be obtained. Then, the state of the key in the key monitoring image may be recognized based on an image segmentation operation, or other image recognition algorithms. Finally, the key control information may be obtained. In the scanning room, the medical staff may operate the medical device based on a key on the gantry, and a state of the key (e.g., pressing, rebounding, or the key being stuck) on the gantry may be monitored by the second monitoring device in the scanning room.

In some embodiments, the state of the key may be recognized by an image recognition algorithm. For example, a training set consisting of a plurality of key images and key images marked with specific states may be used to obtain an image recognition algorithm (i.e., model) for recognizing the state of the key after in-depth learning and training.

In some embodiments, the current operation information of the medical device may be obtained at any time from the object entering the scanning room to the object leaving the scanning room. In some embodiments, the current operation information of the medical device may be obtained after starting the medical device.

In some embodiments, the first monitoring device or the second monitoring device may include at least one of: an image acquisition device, a sensor, or a radar. In some embodiments, both the first monitoring device and the second monitoring device may include an image acquisition device. In some embodiments, the first monitoring device and the second monitoring device may independently include auxiliary detection devices, such as a sensor and/or a radar. For example, the sensor may include an infrared sensor or a sound sensor, and the radar may be a millimeter wave radar. In some embodiments, if an unclear image exists in the current operation information or actual running information obtained by the image acquisition device, an image acquired by the sensor and/or the radar may be used to replace the unclear image, thus the monitoring may be more accurate.

In some embodiments, the actual running information may include image information and sound information. In some embodiments, the actual running information of the medical device may be determined by identifying the image information and sound information in the video.

In some embodiments, the image information in the video may be recognized by the image recognition algorithm, and the sound information in the video may be recognized by the voice recognition algorithm. In some embodiments, the image recognition algorithm and the voice recognition algorithm may be deep-learning algorithms. For example, during training, a running image/ of the medical device for training and a running image marked with the actual running information may be designated as a training set, and in-depth model training may be performed based on the training set to obtain the image recognition algorithm that used to recognize the actual running information. As another example, during training, sound information of the medical device for training and sound information marked with the actual running infomlation may be designated as a training set, and in-depth model training may be performed based on the training set to obtain the sound recognition algorithm that used to recognize the actual running information.

Since there may be an interference involving between other object and the medical device or the gantry when the abnormal motion exists, the medical device may generate different sounds from a sound generated in normal motion, so the abnormal state of the medical device may be determined based on the sound information.

In some embodiments of the present disclosure, the accuracy of recognizing the actual running information may be improved by identifying the image information and sound information of the medical device in the video.

In 830, the running state of the medical device may be controlled based on the actual running information and the expected running information. In some embodiments, operation 830 may be performed by the control module 430 (e.g., the running control unit 434).

The expected running information may refer to a motion corresponding to the current operation information that the medical device should perform in a condition that the medical device is not in an abnormal state. The expected running information may include a tilt of the gantry, a motion of the examination table, or the like.

In some embodiments, the excepted operation information may include a standard operation process.

The standard operation process may refer to operations to be performed and a sequence of each operation in the process of scanning the object, for example, inputting patient information->>moving the examination table->>selecting the scanning protocol->>determining parameters of scanning->>starting to scan->>ending the scan->>processing an image, or the like.

In different stages of scanning, different operations need to be performed, and the expected running information of the corresponding medical device may be different. For example, in an early stage of scanning, when the object is on the examination table, an instruction of adjusting the height of the examination table need to be executed, such as the expected running information of the medical device is moving the examination table up or down. If the actual running information is inconsistent with the expected running information at a corresponding stage, an operation of the medical staff may violate the standard operation process or the medical device may be in an abnormal state.

In some embodiments, the actual running information of the medical device may be compared with the expected running information, and the operation state of the medical device may be obtained after comparing the actual running information and the expected running information.

The running state of the medical device may be configured to evaluate whether the medical device is in an abnormal state, indicating whether there is an abnormal situation in running of medical device. For example, the running state of a medical device may include a normal state or an abnormal state. When the actual running information is consistent with the expected running information, the medical device may be determined in a normal state. At this time, the medical device may maintain the current running state, and the first monitoring device may continue to monitor the medical device. When the actual running information is inconsistent with the expected running information, the medical device may be determined in an abnormal state.

In some embodiments, when the expected running information is inconsistent with the actual running information, the medical device may be determined in an abnormal state. In some embodiments, if the expected running information determined based on the current operation information is inconsistent with the actual running information, the medical device may be determined in an abnormal state, and the current operation information may include internal control information and/or key control information.

In some embodiments, the actual running information of the medical device may be compared with the expected running information corresponding to the key being stuck to determine whether the medical device is in an abnormal state. For example, the medical device may be considered to be in an abnormal state when the key is stuck or there is no person to operate but the medical device is still running.

In some embodiments, when the key control information indicates that the key is stuck, the expected running information corresponding to the key being stuck may be consistent with the expected running information corresponding to the key being rebound. Specifically, when the medical device is not in an abnormal state, the key may rebound normally after being pressed. At this time, the medical device may have a corresponding expected running information. Therefore, if the key cannot rebound normally due to the key being stuck, the medical device may not operate with the expected running information.

In some embodiments of the present disclosure, a special situation of the key being stuck may be considered, and the corresponding expected running information may be set for the key being stuck to meet requirements of different scenarios. In some embodiments, other excepted operation information may be set for the key being stuck based on the actual requirements. For example, when the key is stuck, the medical device may run based on an existing running state, or stop running and remain stationary.

In some embodiments, because the medical device may have abnormal motion caused by a component failure, a software error, or the like monitoring may be performed based on the internal control information, which is a software control instruction received inside the controller. For example, the controller may compare the monitoring image acquired by the first monitoring device with the internal control information of the medical device. If the actual running information obtained from the monitoring image conflicts with the internal control information of the medical device, the medical device may be in an abnormal state.

The key being stuck may include two conditions as follows: 1) The key may be stuck when a medical staff is not operating the medical device; 2) after the medical staff actively presses the key, the key may not be rebounded. Both of the two conditions may cause an abnormal state of the medical device. For the first condition, the actual running information of the medical device may be obtained by the first monitoring device. If the medical device moves without touching the key artificially, the key may be stuck. For the second condition, the monitoring image of the key may be obtained by the second monitoring device, and then the key control information may be obtained from the monitoring image of the key to determine whether the key is rebounded. If the key is not rebounded, the key may be stuck.

It should be noted that if the current operation information is only internal control information or key control information. the current operation information may be determined based on the internal control information or key control information. If the internal control information and key control information are obtained at the same time, and current operation information obtained by the internal control information and key control information respectively are inconsistent, one of the corresponding current operation information may be selected based on a predetermined priority for judgment.

The excepted running information may be obtained based on the internal control information and/or key control information in some embodiments of the present disclosure. Then, the excepted running information may be compared with the actual running information to judge the running state of the medical device, thus the accuracy of judging the running state of the medical device may be improved.

In some embodiments, the current operation information of the medical device may be compared with the excepted running information to determine whether the medical device is in an abnormal state. In some embodiments, the current operation information and previous operation information may be compared with the excepted running information corresponding to previous process, and compared with the excepted running information corresponding to current process to determine whether the medical device is in an abnormal state. For example, if the current operation information is determining a scanning dose, and corresponding excepted running information is determining a scanning protocol, thus the medical device may be in an abnormal state.

In some embodiments, in response to determining that the medical device is in an abnormal state, the operation of the medical device may be stopped, and warning information may be generated based on an abnormal state to remind the medical staff that the medical device has been stopped urgently. Thus, more serious accidents may be avoided and losses may be reduced. In some embodiments, a termination instruction may be sent to the medical device by a controller connected with the medical device to stop the medical device. In some embodiments, the warning information may be generated based on the abnormal state and sent to a communication device of the medical staff, or an audible and visual alarm may be performed.

The actual running information of the medical device may be obtained in real time based on the first monitoring device in some embodiments of the present disclosure, and the actual running information may be compared with the expected information to obtain real-time running state of the medical device, the abnormal motion may be detected at the moment of the medical device is in the abnorm al state, thus the detection efficiency and detection accuracy of the abnormal motion of the medical device may be improved.

FIG. 9 is a flowchart illustrating an exemplary process for obtaining expected running information of a medical device according to some embodiments of the present disclosure.

In some embodiments, a process 900 may be performed by the monitoring system 100 or processing device 400. For example, the process 900 may be stored in a storage device (e.g., the storage device 150) in the form of programs or instructions. The process 900 may be implemented when the monitoring system 100 (e.g., the terminal 130) executes the programs or instructions. The operations of the process 900 presented below is illustrative. In some embodiments, a process of the process 900 may be implemented with one or more additional operations not described and/or one or more operations not discussed. In addition, the order of operations of the process 900 shown in FIG. 9 and described below is not intended to be restrictive.

In 910, current operation information of the medical device may be obtained.

In some embodiments, internal control information of the medical device may be acquired by a controller connected with the medical device and designated as the current operation information. In some embodiments, key control information of the medical device may be obtained by the second monitoring device and designated as the current operation information. More descriptions of obtaining the current operation infonnation may be found elsewhere in the present disclosure, for example, FIG. 8 and the descriptions thereof, which may not be described herein.

In 920, a predetermined correspondence between the current operation information and the excepted running information may be obtained.

For any operation information sent to the medical device, the medical device may have a corresponding feedback, (i.e., the running information), and the correspondence between the operation information and the running information may be stored in the controller in advance.

Scans for the same part or the same type of scans may correspond to the same standard operation process. For example, a scan for the chest and a scan for the abdomen may correspond to different standard operation processes. In the standard operation process, different stages may correspond to different operation information, and correspondingly, may correspond to different excepted running information.

Before monitoring the running state of medical device, correspondence between all operation information and running information may be obtained. For example, under what kind of internal control information and key control information, the examination table may need to move forward accordingly. Under what kind of internal control information and key control information, the examination table needs to go back. Under what kind of key control information, the medical device needs to stop running,

In some embodiments, the predetermined correspondence between the current operation information and the excepted running information may be obtained from a storage device (e.g., the storage device 150).

In 930, excepted running information may be obtained based on the predetermined correspondence and the current operation information.

In some embodiments, after obtaining the current operation information of the medical device, operation information consistent with the current operation information may be found in the predetermined correspondence, and then corresponding running information may be found as the excepted running information based on the predetermined correspondence.

In some embodiments of the present disclosure, determining the excepted running information based on the predetermined correspondence may improve the accuracy of identifying the excepted running information.

It should be noted that the above description of the process 800 and the process 900 is merely for illustration, and does not limit the scope of the present disclosure. For those skilled in the art, various modifications and changes may be made to the process 800 and/or the process 900 under the guidance of the present disclosure. However, these modifications and changes are still within the scope of the present disclosure.

FIG. 10 is a schematic diagram illustrating an exemplary system for monitoring a medical device according to some embodiments of the present disclosure.

As shown in FIG. 10, in some embodiments, a monitoring system 1000 may include the medical device 110 and the console 1020. The console may further include a controller (e.g., the processing device 140) and a key (e.g., the terminal 130). The medical device 110 may be arranged in the scanning room 101, and the console 1020 may be arranged in the operation room 102.

In some embodiments, the first monitoring device 160-1 may be arranged in the scanning room 101 to monitor the medical device 110, and the second monitoring device 160-2 may be arranged in the operation room 102 or the scanning room 101 to monitor one or more keys of the console 1020 and one or more keys on the gantry of the medical device 110.

In some embodiments, the controller may be composed of one or more central processors, which may be configured to receive signals such as the actual running information of the medical device 110 acquired by the first monitoring device 160-1 and the key control information acquired by the second monitoring device 160-2. The controller may be configured to receive the internal control information of the medical device 110.

In some embodiments, the one or more keys may include a virtual touch-screen key and/or a physical key.

In some embodiments, a count of the first monitoring device 160-1 and a count of the second monitoring device 160-2 may be determined based on actual requirements. It should be noted that a position and the count of the first monitoring device 160-1 and t a position and the count of the second monitoring device 160-2 in FIG. 10 are merely for illustration and not be limited.

In the process of monitoring medical device, taking a CT system as an example, the key control information may include whether there is a medical staff operating the console 1020, a CTBOX, a control panel on the gantry of the CT system, or the like. The CTBOX is a control box of the CT system. When the CT system moves, the controller may compare the actual running information in the scanning room 101 acquired by the first monitoring device 160-1 with the internal control information to determine whether a motion of the CT system is triggered artificially, purposefully and consciously. When an abnormal motion of the CT system exists, the CT system may be in an abnormal state, and the controller may terminate the abnormal motion of the CT system, and prompt a staff that the abnormal motion (e.g., the key being stuck) exists based on alarms, or other ways.

In some embodiments, when the controller performs a processing operation, the image information and sound information of the operation room 102 and the scanning room 101 may be analyzed. Specifically, the image information may refer to the actual running information acquired by the first monitoring device 160-1 or the current operation information acquired by the second monitoring device 160-2. The sound information may be a sound (e.g., a sound of the examination table hitting on the gantry) of the CT system in the abnormal state in the scanning room 101. In some embodiments, the image information and the sound information may be obtained at different times.

In some embodiments, the controller may fuse the image information in the operation room and the image information scanning room at the same time to make a more accurate comparison.

In some embodiments, the controller may fuse the image information, the sound information and data information acquired by a detection device, such as a line sensor, a radar, or the like.

The abnormal motion of the medical device 110 may be detected based on the first monitoring device 160-1 and the second monitoring device 160-2 in the embodiment, and the abnormal motion may be detected and warned immediately when the medical device is in an abnormal state. At the same time, when the medical device is in an abnormal state, a motion of the medical device may be stopped immediately. Thus, the risks may be reduced. Thus, a real-time running performance of the medical device may be monitored accurately, and the abnormal state of the medical device may be quickly detected and determined, thus the abnormal state may be timely terminated to avoid harm and the safety of the medical device may be ensured.

It should be noted that those skilled in the art may make various reasonable changes to the technical scheme of the present disclosure. For example, a count of cameras, an arrangement of the cameras and/or a location of cameras in the first monitoring device 160-1 and the second monitoring device 160-2 may be determined based on actual requirements. For example, the arrangement of cameras may include, but be not limited to: array arrangement, ring arrangement, topology arrangement, or the like. These changes are still within the scope of the present disclosure.

According to some embodiments of the present disclosure, (1) the monitoring device may be configured to acquire the posture information of the object, and the height of the examination table may be adjusted based on the posture information, thus the convenience and efficiency of medical scanning may be improved; (2) a pre-start operation of the medical device may be performed based on a corresponding scanning protocol of the identity information of the object, thus the scanning time of the scanning may be reduced, and the scanning may be performed efficiently; (3) an actual positioning of the object may be identified based on the second image acquired by the monitoring device and the positioning of the object may be guided based on the actual positioning, thus the object may be helped to be in an appropriate positioning state, and the scanning efficiency may be improved; (4) by simulating a motion trajectory of the examination table from a position where the object goes to the examination table to the target position, an interference involved between the object and the medical device may be avoided and the safety of the object m ay be improved; (5) by acquiring the mental state information of the object by the monitoring device and judging whether it is suitable for scanning based on the mental state information, a condition that the poor mental state of the object may affect a scanning result may be avoided, and a secondary injury caused by the scanning to the object may also be avoid; (6) based on the actual running information and excepted running information of medical device, whether the medical device is in an abnormal state may be determined, and a corresponding control operation may be performed, thus the safety may be improved; (7) when an abnormal object exists or the object is not in the predetermined region, the warning information may be generated, thus an interference of irrelevant person to the scanning may be reduced, and the scanning efficiency may be improved. It should be noted that different embodiments may have different beneficial effects. In different embodiments, the possible beneficial effects may be any one of the above, or a combination thereof, or any other possible beneficial effects.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the scope of the exemplary embodiments of this disclosure.

Meanwhile, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the scope of the disclosed embodiments. For example, although the implementation of various components described above may be embodied in a hardware device, it may also be implemented as a software only solution-e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that the present disclosure object requires more features than the features mentioned in the claims. Rather, claimed subject matter may lie in less than all features of a single foregoing disclosed embodiment.

In some embodiments, the numbers expressing quantities of ingredients, properties, and so forth, used to describe and claim certain embodiments of the application are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Finally, it should be understood that the embodiments described in the present disclosure merely illustrates the principles of the embodiments of the present disclosure. Other modifications may be within the scope of the present disclosure. Accordingly, by way of example, and not limitation, alternative configurations of embodiments of the present disclosure may be considered to be consistent with the teachings of the present disclosure. Accordingly, the embodiments of the present disclosure are not limited to the embodiments explicitly introduced and described by the present disclosure.

## Claims

1. A method for monitoring a medical device (110), comprising:
obtaining a plurality of first images of a scanning room (101) acquired by a first monitoring device (160-1) arranged in the scanning room (101);
obtaining an analysis result by analyzing the plurality of first images, wherein each of at least a portion of the plurality of first images includes an object, controlling the medical device based on the analysis result, the medical device including an examination table; wherein a control mode of the medical device includes at least one of controlling the medical device to adjust a height of the examination table to a target height based on the posture information of the object the method **characterized in that** the
analysis result includes posture information of the object, the posture information includes a sitting posture, a standing posture, or a lying posture, and the posture information is determined by:
determining feature points of a body of the object based on the plurality of first images;
determining whether the object is in the lying posture based on information of the feature points; and
determining whether the object is in the sitting posture or the standing posture based on leg joints of the object when determining that the body of
the object is in an upright state based on the information of the feature points; and **in that** the control mode includes:
in response to determining that the object walks into the scanning room (101), obtaining a length of a leg of the object; and lowering the height of the examination table (116) when the object goes to the examination table (116) to below the length of the leg of the object;
in response to determining that the object enters into the scanning room (101) with a wheelchair, obtaining a height of the wheelchair; and lowering the height of the examination table (116) when the object goes to the examination table (116) to below the height of the wheelchair; or
in response to determining that the object enters into the scanning room (101) on a stretcher, obtaining a height of the stretcher, and lowering the height of the examination table (116) when the object goes to the examination table (116) to below the height of the stretcher.

2. The method of claim 1, wherein the determining whether the object is in the sitting posture or the standing posture based on leg joints of the object includes:
connecting a knee joint with an ankle joint by a line segment;
connecting the knee joint with a cross joint by another line segment based on coordinates of each joint of the leg joints; and
determining whether the object is in the sitting posture or the standing posture based on an angle between the two line segments.

3. The method of claim 1, wherein the control mode of the medical device (110) further includes controlling the medical device (110) to send the object to a target position by:
obtaining a scanning protocol based on identity information of the object, wherein the scanning protocol includes predetermined positioning information;
obtaining a plurality of second images of the object on the examination table (116) acquired by the first monitoring device (160-1) when the object is on the examination table (116);
determining actual positioning information of the object based on the plurality of second images;
controlling the medical device (110) to send the object to the target position for a scan in response to determining that the actual positioning information meets a predetermined condition by comparing the actual positioning information with the predetermined positioning information; and
adjusting an actual positioning of the object on the examination table (116) in response to determining that the actual positioning information does not meet the predetermined condition by comparing the actual positioning information with the predetermined positioning information.

4. The method of claim 3, wherein the scanning protocol includes a reference rotation speed of a gantry of the medical device (110), one or more reference parameters associate with anode preheating of a tube of the medical device (110), and a reference positioning state of each of one or more components of the medical device (110), before adjusting the height of the examination table (116), the method includes at least one of operations including:
adjusting a rotation speed of the gantry of the medical device (110) to the rotation speed in the scanning protocol, adjusting one or more parameters associated with anode preheating of the tube of the medical device (110) to the one or more reference parameters associate with anode preheating in the scanning protocol, and adjusting a positioning state of each of the one or more components of the medical device (110) to the reference positioning state in the scanning protocol.

5. The method of claim 3 or 4, the method further comprising:
before sending the object to the target position,
simulating a motion trajectory of the examination table (116) from a position where the object goes to the examination table (116) to the target position; and
determining whether an interference involves between the object and the medical device (110) based on the motion trajectory.

6. The method of any one of claims 3-5, wherein sending the object to the target position for a scan in response to determining that the actual positioning information meets a predetermined condition by comparing the actual positioning information with the predetermined positioning information includes:
determining a scanning dose of the object based on the actual positioning information;
sending the object to the target position for the scan in response to determining that the scanning dose meets a requirement.

7. The method of any one of claims 1-6, wherein the plurality of first images includes a video of the medical device (110), the analysis result includes actual running information of the medical device (110) and controlling the medical device (110) based on an analysis result includes controlling a running state of the medical device (110) by:
controlling the running state of the medical device (110) based on the actual running information and expected running information, wherein the actual running information includes image information and sound information.

8. The method of claim 7, further including:
obtaining current operation information of the medical device (110); and
determining the expected running information of the medical device (110) based on the current operation information.

9. The method of claim 8, wherein obtaining current operation information of the medical device (110) includes at least one of:
designating internal control information of the medical device (110) as the current operation information, the internal control information being acquired based on a controller connected with the medical device (110); or
designating key control information of the medical device (110) as the current operation information, the key control information being acquired based on a second monitoring device (160-2).

10. The method of claim 9, wherein when the key control information indicates that a key being stuck, the expected running information corresponding to the key being stuck and the expected running information corresponding to the key being rebounded are consistent.

11. The method of any one of claims 8-10, wherein the determining the expected running information of the medical device (110) based on the current operation information includes:
obtaining a predetermined correspondence between the current operation information and the excepted running information; and
determining the excepted running information based on the predetermined correspondence and the current operation information.

12. The method of any one of claims 7-11, wherein the controlling the running state of the medical device (110) based on the actual running information and the expected running information includes:
in response to determining that the expected running information is inconsistent with the actual running information, determining that the medical device (110) is in an abnormal state; and
sending a motion termination instruction to the medical device (110) via a controller connected with the medical device (110); and
generating warning information.

13. A medical device (110), comprising:
a scanner configured to perform a medical scan to obtain scanning data of a scanning region of an object;
an image acquisition device configured to obtain a plurality of first images of a scanning room (101);
a processor configured to obtain an analysis result by analyzing the plurality of first images and determine a control mode of the medical device (110) based on the analysis result, wherein each of at least a portion of the plurality of first images includes the object, the analysis result includes posture information of the object,
the processor further configured to implement a control mechanism to control the medical device (110) based on the control mode, wherein the medical device (110) includes an examination table (116), the control mode of the medical device (110) includes controlling the medical device (110) to adjust a height of the examination table (116) to a target height based on the posture information of the object, the medical device **characterized in that** the processor is further configured to determine the posture information as a sitting posture, a standing posture, or a lying posture, and the posture information is determined by:
determining feature points of a body of the object based on the plurality of first images;
determining whether the object is in the lying posture based on information of the feature points; and
determining whether the object is in the sitting posture or the standing posture based on leg joints of the object when determining that the body of the object is in an upright state based on the information of the feature points; and
and **in that** the control mechanism implemented by the processor further includes:
in response to determining that the object walks into the scanning room (101), obtaining a length of a leg of the object; and lowering the height of the examination table (116) when the object goes to the examination table (116) to below the length of the leg of the object;
in response to determining that the object enters into the scanning room (101) with the wheelchair, obtaining a height of the wheelchair; and lowering the height of the examination table (116) when the object goes to the examination table (116) to below the height of the wheelchair; or
in response to determining that the object enters into the scanning room (101) on a stretcher, obtaining a height of the stretcher, and lowering the height of the examination table (116) when the object goes to the examination table (116) to below the height of the stretcher.

14. A system for monitoring a medical device (110), comprising:
an acquisition module configured to obtain a plurality of first images of a scanning room (101) acquired by a first monitoring device (160-1) arranged in the scanning room (101);
an image analysis module configured to obtain an analysis result by analyzing the plurality of first images, wherein each of at least a portion of the plurality of first images includes an object, the analysis result includes posture information of the object, a control module configured to control the medical device (110) based on the analysis result, the medical device (110) including an examination table (116)
the system **characterized in that** the image analysis module obtains an analysis result as the posture information including a sitting posture, a standing posture, or a lying posture, and the posture information is determined by:
determining feature points of a body of the object based on the plurality of first images;
determining whether the object is in the lying posture based on information of the feature points; and
determining whether the object is in the sitting posture or the standing posture based on leg joints of the object when determining that the body of the object is in an upright state based on the information of the feature points; and **in that** the control module implements a control mode of the medical device (110) that includes controlling the medical device (110) to adjust a height of the examination table (116) to a target height based on the posture information of the object, including:
in response to determining that the object walks into the scanning room (101), obtaining a length of a leg of the object; and lowering the height of the examination table (116) when the object goes to the examination table (116) to below the length of the leg of the object;
in response to determining that the object enters into the scanning room (101) with the wheelchair, obtaining a height of the wheelchair; and lowering the height of the examination table (116) when the object goes to the examination table (116) to below the height of the wheelchair; or
in response to determining that the object enters into the scanning room (101) on a stretcher, obtaining a height of the stretcher, and lowering the height of the examination table (116) when the object goes to the examination table (116) to below the height of the stretcher.

15. The method of claim 5, the method further comprising:
before sending the object to the target position,
determining an interference region corresponding to the examination table (116) based on the examination table (116) and the motion trajectory from a position where the object goes to the examination table motion trajector to the target position,
determining whether an interference involves between the object and one or more components of the medical device (110) based on the interference region;
in response to the interference does not involve between the object and the one or more components, sending the object to the target position for a scan; and
in response to the interference involves between the object and the one or more components, adjusting a scanning posture of the object to eliminate a risk of the interference.

## Patentansprüche

1. Verfahren zum Überwachen einer medizinischen Vorrichtung (110), umfassend:
Erhalten einer Vielzahl von ersten Bildern eines Untersuchungsraums (101), welche von einer in dem Untersuchungsraum (101) angeordneten ersten Überwachungsvorrichtung (160-1) erfasst werden;
Erhalten eines Analyseergebnisses durch Analysieren der Vielzahl von ersten Bildern, wobei zumindest ein Abschnitt der Vielzahl von ersten Bildern jeweils ein Objekt beinhaltet, Steuern der medizinischen Vorrichtung basierend auf dem Analyseergebnis, wobei die medizinische Vorrichtung eine Untersuchungsliege beinhaltet; wobei ein Steuerungsmodus der medizinischen Vorrichtung zumindest eines der folgenden beinhaltet: Steuern der medizinischen Vorrichtung, um eine Höhe der Untersuchungsliege basierend auf den Haltungsinformationen des Objekts auf eine Zielhöhe anzupassen, wobei das Verfahren **dadurch gekennzeichnet ist, dass** das Analyseergebnis Haltungsinformationen des Objekts beinhaltet, wobei die Haltungsinformationen eine Sitzhaltung, eine Stehhaltung oder eine Liegehaltung beinhalten, und die Haltungsinformationen bestimmt werden durch:
Bestimmen von Merkmalspunkten eines Körpers des Objekts basierend auf einer Vielzahl von ersten Bildern;
Bestimmen, ob sich das Objekt in der Liegehaltung befindet, basierend auf den Informationen über die Merkmalspunkte; und
Bestimmen, ob sich das Objekt in der Sitzhaltung oder der Stehhaltung befindet, basierend auf Beingelenken des Objekts, wenn basierend auf den Informationen der Merkmalspunkte bestimmt wird, dass sich der Körper des Objekts in einer aufrechten Position befindet;
und dass der Steuerungsmodus beinhaltet:
als Reaktion auf Bestimmen, dass das Objekt im Untersuchungsraum (101) geht, Erhalten einer Länge eines Beins des Objekts; und Absenken der Höhe der Untersuchungsliege (116) auf unter die Länge des Beins des Objekts, wenn sich das Objekt zur Untersuchungsliege (116) begibt;
als Reaktion auf Bestimmen, dass das Objekt mit einem Rollstuhl in den Untersuchungsraum (101) kommt, Erhalten einer Höhe des Rollstuhls; und Absenken der Höhe der Untersuchungsliege (116) auf unter die Höhe des Rollstuhls, wenn sich das Objekt zur Untersuchungsliege (116) begibt; oder
als Reaktion auf Bestimmen, dass das Objekt auf einer Trage in den Untersuchungsraum (101) kommt, Erhalten einer Höhe der Trage, und Absenken der Höhe der Untersuchungsliege (116) auf unter die Höhe der Trage, wenn sich das Objekt zur Untersuchungsliege (116) begibt.

2. Verfahren nach Anspruch 1, wobei das Bestimmen, ob sich das Objekt in der Sitzhaltung oder der Stehhaltung befindet, basierend auf Beingelenken des Objekts, beinhaltet:
Verbinden eines Kniegelenks mit einem Sprunggelenk durch eine Strecke;
Verbinden des Kniegelenks mit einem Kreuzgelenk durch eine weitere Strecke, basierend auf Koordinaten jedes Gelenks der Beingelenke; und
Bestimmen, ob sich das Objekt in der Sitzhaltung oder der Stehhaltung befindet, basierend auf einem Winkel zwischen den zwei Strecken.

3. Verfahren nach Anspruch 1, wobei der Steuerungsmodus der medizinischen Vorrichtung (110) weiter Steuern der medizinischen Vorrichtung (110) zum Bewegen des Objekts in eine Zielposition beinhaltet, durch:
Erhalten eines Scan-Protokolls basierend auf Identitätsinformationen des Objekts, wobei das Scan-Protokoll vorbestimmte Positionsinformationen beinhaltet;
Erhalten einer Vielzahl von zweiten Bildern des Objekts auf der Untersuchungsliege (116), welche von der ersten Überwachungsvorrichtung (160-1) erfasst wurden, wenn sich das Objekt auf der Untersuchungsliege (116) befindet;
Bestimmen von tatsächlichen Positionsinformationen des Objekts basierend auf der Vielzahl von zweiten Bildern;
Steuern der medizinischen Vorrichtung (110) zum Bewegen des Objekts in die Zielposition für einen Scan, als Reaktion auf Bestimmen, dass die tatsächlichen Positionsinformationen eine vorbestimmte Bedingung erfüllen, durch Vergleichen der tatsächlichen Positionsinformationen mit den vorbestimmten Positionsinformationen; und
Anpassen einer tatsächlichen Positionierung des Objekts auf der Untersuchungsliege (116) als Reaktion auf Bestimmen, dass die tatsächlichen Positionsinformationen die vorbestimmten Bedingung nicht erfüllen, durch Vergleichen der tatsächlichen Positionsinformationen mit den vorbestimmten Positionsinformationen.

4. Verfahren nach Anspruch 3, wobei das Scan-Protokoll eine Referenzrotationsgeschwindigkeit eines Portals der medizinischen Vorrichtung (110), einen oder mehrere Referenzparameter im Zusammenhang mit Anodenvorwärmung einer Röhre der medizinischen Vorrichtung (110), und einen Referenzpositionszustand jeder von einer oder mehreren Komponenten der medizinischen Vorrichtung (110) vor Anpassen der Höhe der Untersuchungsliege (116) beinhaltet, das Verfahren zumindest einen der Vorgänge beinhaltet, welche beinhalten:
Anpassen der Rotationsgeschwindigkeit des Portals der medizinischen Vorrichtung (110) an die Rotationsgeschwindigkeit im Scan-Protokoll, Anpassen eines oder mehrerer Parameter im Zusammenhang mit der Anodenvorwärmung der Röhre der medizinischen Vorrichtung (110) an den einen oder die mehreren Referenzparameter im Zusammenhang mit der Anodenvorwärmung im Scan-Protokoll, und Anpassen eines Positionierungszustands jeder der einen oder mehreren Komponenten der medizinischen Vorrichtung (110) an den Referenzpositionszustand im Scan-Protokoll.

5. Verfahren nach Anspruch 3 oder 4, weiter umfassend:
bevor das Objekt in die Zielposition bewegt wird,
Simulieren einer Bewegungsbahn der Untersuchungsliege (116) von einer Position, in welcher sich das Objekt zur Untersuchungsliege (116) begibt, zur Zielposition; und
Bestimmen, ob eine Interferenz zwischen dem Objekt und der medizinischen Vorrichtung (110) vorliegt, basierend auf der Bewegungsbahn.

6. Verfahren nach einem der Ansprüche 3-5, wobei Bewegen des Objekts in die Zielposition für einen Scan, als Reaktion auf Bestimmen, dass die tatsächlichen Positionsinformationen eine vorbestimmte Bedingung erfüllen, durch Vergleichen der tatsächlichen Positionsinformationen mit den vorbestimmten Positionsinformationen beinhaltet:
Bestimmen einer Scan-Dosis des Objekts basierend auf den tatsächlichen Positionsinformationen;
Bewegen des Objekts in die Zielposition für den Scan, als Reaktion auf Bestimmen, dass die Scan-Dosis eine Anforderung erfüllt.

7. Verfahren nach einem der Ansprüche 1-6, wobei die Vielzahl von ersten Bildern ein Video der medizinischen Vorrichtung (110) beinhaltet, das Analyseergebnis tatsächliche Betriebsdaten der medizinischen Vorrichtung (110) beinhaltet, und Steuern der medizinischen Vorrichtung (110) basierend auf einem Analyseergebnis Steuern eines Betriebszustands der medizinischen Vorrichtung (110) beinhaltet, durch:
Steuern des Betriebszustands der medizinischen Vorrichtung (110) basierend auf den tatsächlichen Betriebsdaten und erwarteten Betriebsdaten, wobei die tatsächlichen Betriebsdaten Bildinformation und Toninformationen beinhalten.

8. Verfahren nach Anspruch 7, weiter beinhaltend:
Erhalten von aktuellen Betriebsinformationen der medizinischen Vorrichtung (110); und
Bestimmen der erwarteten Betriebsdaten der medizinischen Vorrichtung (110) basierend auf den aktuellen Betriebsinformationen.

9. Verfahren nach Anspruch 8, wobei Erhalten von aktuellen Betriebsinformationen der medizinischen Vorrichtung (110) zumindest eines der folgenden beinhaltet:
Bezeichnen von internen Steuerungsinformationen der medizinischen Vorrichtung (110) als die aktuelle Betriebsinformationen, wobei die internen Steuerungsinformationen basierend auf einer mit der medizinischen Vorrichtung (110) verbundenen Steuereinheit erfasst werden; oder
Bezeichnen von wichtigen Steuerungsinformationen der medizinischen Vorrichtung (110) als die aktuellen Betriebsinformationen, wobei die wichtigen Steuerungsinformationen basierend auf einer zweiten Überwachungsvorrichtung (160-2) erfasst werden.

10. Verfahren nach Anspruch 9, wobei, wenn die wichtigen Steuerungsinformationen anzeigen, dass eine Taste klemmt, die erwarteten Betriebsdaten, welche der klemmenden Taste entsprechen, und die erwarteten Betriebsdaten, welche der zurückprallenden Taste entsprechen, übereinstimmen.

11. Verfahren nach einem der Ansprüche 8-10, wobei das Bestimmen der erwarteten Betriebsdaten der medizinischen Vorrichtung (110) basierend auf den aktuellen Betriebsinformationen beinhaltet:
Erhalten einer vorbestimmten Übereinstimmung zwischen den aktuellen Betriebsinformationen und den erwarteten Betriebsdaten; und
Bestimmen der erwarteten Betriebsdaten basierend auf der vorbestimmten Übereinstimmung und den aktuellen Betriebsinformationen.

12. Verfahren nach einem der Ansprüche 7-11, wobei das Steuern des Betriebszustands der medizinischen Vorrichtung (110) basierend auf den tatsächlichen Betriebsdaten und den erwarteten Betriebsdaten beinhaltet:
als Reaktion auf Bestimmen, dass die erwarteten Betriebsdaten nicht mit den tatsächlichen Betriebsdaten übereinstimmen, Bestimmen, dass sich die medizinische Vorrichtung (110) in einem anormalen Zustand befindet; und
Senden einer Bewegungsbeendigungsanweisung über eine mit der medizinischen Vorrichtung (110) verbundene Steuereinheit an die medizinische Vorrichtung (110); und
Generieren von Warnmeldungen.

13. Medizinische Vorrichtung (110), umfassend:
einen Scanner, welcher dazu konfiguriert ist, einen medizinischen Scan durchzuführen, um Scan-Daten eines Scan-Bereichs eines Objekts zu erhalten;
eine Bilderfassungsvorrichtung, welche dazu konfiguriert ist, eine Vielzahl von ersten Bildern eines Untersuchungsraums (101) zu erhalten;
einen Prozessor, welcher dazu konfiguriert ist, ein Analyseergebnis durch Analysieren der Vielzahl von ersten Bildern zu erhalten und basierend auf dem Analyseergebnis einen Steuerungsmodus der medizinischen Vorrichtung (110) zu bestimmen, wobei zumindest ein Abschnitt der Vielzahl von ersten Bildern das Objekt beinhaltet, das Analyseergebnis Haltungsinformationen des Objekts beinhaltet;
wobei der Prozessor weiter dazu konfiguriert ist, einen Steuerungsmechanismus zum Steuern der medizinischen Vorrichtung (110) basierend auf dem Steuerungsmodus zu implementieren, wobei die medizinische Vorrichtung (110) eine Untersuchungsliege (116) beinhaltet, der Steuerungsmodus der medizinischen Vorrichtung (110) Steuern der medizinischen Vorrichtung (110), um eine Höhe der Untersuchungsliege (116) basierend auf den Haltungsinformationen des Objekts auf eine Ziellhöhe anzupassen, beinhaltet, wobei die medizinische Vorrichtung **dadurch gekennzeichnet ist, dass** der Prozessor weiter dazu konfiguriert ist, die Haltungsinformationen als eine Sitzhaltung, eine Stehhaltung oder eine Liegehaltung zu bestimmen, und die Haltungsinformationen bestimmt werden durch:
Bestimmen von Merkmalspunkten eines Körpers des Objekts basierend auf einer Vielzahl von ersten Bildern;
Bestimmen, ob sich das Objekt in der Liegehaltung befindet, basierend auf den Informationen über die Merkmalspunkte; und
Bestimmen, ob sich das Objekt in der Sitzhaltung oder der Stehhaltung befindet, basierend auf Beingelenken des Objekts, wenn basierend auf den Informationen der Merkmalspunkte bestimmt wird, dass sich der Körper des Objekts in einer aufrechten Position befindet; und
und dass der vom Prozessor implementierte Steuerungsmechanismus weiter beinhaltet:
als Reaktion auf Bestimmen, dass das Objekt im Untersuchungsraum (101) geht, Erhalten einer Länge eines Beins des Objekts; und Absenken der Höhe der Untersuchungsliege (116) auf unter die Länge des Beins des Objekts, wenn sich das Objekt zur Untersuchungsliege (116) begibt;
als Reaktion auf Bestimmen, dass das Objekt mit dem Rollstuhl in den Untersuchungsraum (101) kommt, Erhalten einer Höhe des Rollstuhls; und Absenken der Höhe der Untersuchungsliege (116) auf unter die Höhe des Rollstuhls, wenn sich das Objekt zur Untersuchungsliege (116) begibt; oder
als Reaktion auf Bestimmen, dass das Objekt auf einer Trage in den Untersuchungsraum (101) kommt, Erhalten einer Höhe der Trage, und Absenken der Höhe der Untersuchungsliege (116) auf unter die Höhe der Trage, wenn sich das Objekt zur Untersuchungsliege (116) begibt.

14. System zum Überwachen einer medizinischen Vorrichtung (110), umfassend:
ein Erfassungsmodul, welches dazu konfiguriert ist, eine Vielzahl von ersten Bildern eines Untersuchungsraums (101) zu erhalten, welche von einer in dem Untersuchungsraum(101) angeordneten ersten Überwachungsvorrichtung (160-1) erfasst werden;
ein Bildanalysemodul, welches dazu konfiguriert ist, ein Analyseergebnis durch Analysieren der Vielzahl von ersten Bildern zu erhalten, wobei jeder von zumindest einem Abschnitt der Vielzahl von ersten Bildern ein Objekt beinhaltet, das Analyseergebnis Haltungsinformationen des Objekts beinhaltet; ein Steuerungsmodul, welches dazu konfiguriert ist, die medizinische Vorrichtung (110) basierend auf dem Analyseergebnis zu steuern, die medizinische Vorrichtung (110) eine Untersuchungsliege (116) beinhaltet, wobei das System **dadurch gekennzeichnet ist, dass** das Bildanalysemodul ein Analyseergebnis erhält, wobei die Haltungsinformationen eine Sitzhaltung, eine Stehhaltung oder eine Liegehaltung beinhalten, und die Haltungsinformationen bestimmt werden durch:
Bestimmen von Merkmalspunkten eines Körpers des Objekts basierend auf einer Vielzahl von ersten Bildern;
Bestimmen, ob sich das Objekt in der Liegehaltung befindet, basierend auf den Informationen über die Merkmalspunkte; und
Bestimmen, ob sich das Objekt in der Sitzhaltung oder der Stehhaltung befindet, basierend auf Beingelenken des Objekts, wenn basierend auf den Informationen der Merkmalspunkte bestimmt wird, dass sich der Körper des Objekts in einer aufrechten Position befindet;
und dass das Steuerungsmodul einen Steuerungsmodus der medizinischen Vorrichtung (110) implementiert, welcher Steuern der medizinischen Vorrichtung (110) beinhaltet, um eine Höhe der Untersuchungsliege (116) basierend auf den Haltungsinformationen des Objekts auf eine Zielhöhe anzupassen, beinhaltend:
als Reaktion auf Bestimmen, dass das Objekt im Untersuchungsraum (101) geht, Erhalten einer Länge eines Beins des Objekts; und Absenken der Höhe der Untersuchungsliege (116) auf unter die Länge des Beins des Objekts, wenn sich das Objekt zur Untersuchungsliege (116) begibt;
als Reaktion auf Bestimmen, dass das Objekt mit dem Rollstuhl in den Untersuchungsraum (101) kommt, Erhalten einer Höhe des Rollstuhls; und Absenken der Höhe der Untersuchungsliege (116) auf unter die Höhe des Rollstuhls, wenn sich das Objekt zur Untersuchungsliege (116) begibt; oder
als Reaktion auf Bestimmen, dass das Objekt auf einer Trage in den Untersuchungsraum (101) kommt, Erhalten einer Höhe der Trage, und Absenken der Höhe der Untersuchungsliege (116) auf unter die Höhe der Trage, wenn sich das Objekt zur Untersuchungsliege (116) begibt.

15. Verfahren nach Anspruch 5, wobei das Verfahren weiter umfasst:
bevor das Objekt in die Zielposition bewegt wird,
Bestimmen eines Interferenzbereichs, welcher der Untersuchungsliege (116) entspricht, basierend auf der Untersuchungsliege (116) und der Bewegungsbahn von einer Position, in welcher sich das Objekt zur Bewegungsbahn der Untersuchungsliege begibt, bis zur Zielposition,
Bestimmen, ob eine Interferenz zwischen dem Objekt und einer oder mehreren Komponenten der medizinischen Vorrichtung (110) vorliegt, basierend auf dem Interferenzbereich.
als Reaktion darauf, dass die Interferenz nicht zwischen dem Objekt und der einen oder den mehreren Komponenten vorliegt, Bewegen des Objekts in die Zielposition für einen Scan; und
als Reaktion darauf, dass die Interferenz zwischen dem Objekt und der einen oder mehreren Komponenten vorliegt, Anpassen einer Scan-Haltung des Objekts, um das Risiko einer Interferenz zu eliminieren.

## Revendications

1. Procédé de surveillance d'un dispositif médical (110), comprenant :
l'obtention d'une pluralité de premières images d'une salle de numérisation (101) acquises par un premier dispositif de surveillance (160-1) agencé dans la salle de numérisation (101) ;
l'obtention d'un résultat d'analyse par l'analyse de la pluralité de premières images, dans lequel chacune d'au moins une partie de la pluralité de premières images inclut un objet, la commande du dispositif médical sur la base du résultat d'analyse, le dispositif médical incluant une table d'examen ; dans lequel un mode de commande du dispositif médical inclut au moins une commande du dispositif médical pour ajuster une hauteur de la table d'examen à une hauteur cible sur la base des informations de posture de l'objet, le procédé étant **caractérisé en ce que** le résultat d'analyse inclut des informations de posture de l'objet, les informations de posture incluent une posture assise, une posture debout ou une posture couchée, et les informations de posture sont déterminées par :
la détermination de points caractéristiques d'un corps de l'objet sur la base de la pluralité de premières images ;
la détermination si l'objet est dans la posture couchée sur la base des informations des points caractéristiques ; et
la détermination si l'objet est dans la posture assise ou debout sur la base des articulations des jambes de l'objet lors de la détermination que le corps de l'objet est dans un état vertical sur la base des informations des points caractéristiques ;
et **en ce que** le mode de commande inclut :
en réponse à la détermination que l'objet entre dans la salle de numérisation (101), l'obtention d'une longueur d'une jambe de l'objet ; et l'abaissement de la hauteur de la table d'examen (116) lorsque l'objet se dirige vers la table d'examen (116) en dessous de la longueur de la jambe de l'objet ;
en réponse à la détermination que l'objet entre dans la salle de numérisation (101) avec un fauteuil roulant, l'obtention d'une hauteur du fauteuil roulant ; et l'abaissement de la hauteur de la table d'examen (116) lorsque l'objet se dirige vers la table d'examen (116) en dessous de la hauteur du fauteuil roulant ; ou
en réponse à la détermination que l'objet entre dans la salle de numérisation (101) sur une civière, l'obtention d'une hauteur de la civière et l'abaissement de la hauteur de la table d'examen (116) lorsque l'objet se dirige vers la table d'examen (116) en dessous de la hauteur de la civière.

2. Procédé selon la revendication 1, dans lequel la détermination de la posture assise ou debout de l'objet sur la base des articulations des jambes de l'objet inclut :
la liaison d'une articulation du genou à une articulation de la cheville par un segment de ligne ;
la liaison de l'articulation du genou à une articulation transversale par un autre segment de ligne sur la base de coordonnées de chaque articulation parmi les articulations des jambe ; et
la détermination si l'objet est en posture assise ou debout sur la base d'un angle entre les deux segments de ligne.

3. Procédé selon la revendication 1, dans lequel le mode de commande du dispositif médical (110) inclut en outre la commande du dispositif médical (110) pour envoyer l'objet vers une position cible par :
l'obtention d'un protocole de numérisation sur la base d'informations d'identité de l'objet, dans lequel le protocole de numérisation inclut des informations de positionnement prédéterminées ;
l'obtention d'une pluralité de secondes images de l'objet sur la table d'examen (116) acquises par le premier dispositif de surveillance (160-1) lorsque l'objet se trouve sur la table d'examen (116) ;
la détermination d'informations de positionnement réel de l'objet sur la base de la pluralité de secondes images ;
la commande du dispositif médical (110) pour envoyer l'objet à la position cible pour une numérisation en réponse à la détermination que les informations de positionnement réel remplissent une condition prédéterminée en comparant les informations de positionnement réel aux informations de positionnement prédéterminées ; et
l'ajustement du positionnement réel de l'objet sur la table d'examen (116) en réponse à la détermination que les informations de positionnement réel ne remplissent pas la condition prédéterminée en comparant les informations de positionnement réel aux informations de positionnement prédéterminées.

4. Procédé selon la revendication 3, dans lequel le protocole de numérisation inclut une vitesse de rotation de référence d'un portique du dispositif médical (110), un ou plusieurs paramètres de référence associés au préchauffage d'anode d'un tube du dispositif médical (110), et un état de positionnement de référence de chacun d'un ou plusieurs composants du dispositif médical (110), avant l'ajustement de la hauteur de la table d'examen (116), le procédé inclut au moins une d'opérations incluant :
l'ajustement d'une vitesse de rotation du portique du dispositif médical (110) à la vitesse de rotation dans le protocole de numérisation, l'ajustement d'un ou plusieurs paramètres associés au préchauffage d'anode du tube du dispositif médical (110) au(x) paramètre(s) de référence associé(s) au préchauffage d'anode dans le protocole de numérisation, et l'ajustement d'un état de positionnement de chacun du ou des composants du dispositif médical (110) à l'état de positionnement de référence dans le protocole de numérisation.

5. Procédé selon la revendication 3 ou 4, le procédé comprenant en outre :
avant l'envoi de l'objet à la position cible,
la simulation d'une trajectoire de mouvement de la table d'examen (116) depuis une position où l'objet se dirige vers la table d'examen (116) jusqu'à la position cible ; et
la détermination si une interférence se produit entre l'objet et le dispositif médical (110) sur la base de la trajectoire de mouvement.

6. Procédé selon l'une quelconque des revendications 3-5, dans lequel l'envoi de l'objet à la position cible pour une numérisation en réponse à la détermination que les informations de positionnement réel remplissent une condition prédéterminée en comparant les informations de positionnement réel aux informations de positionnement prédéterminées inclut :
la détermination d'une dose de numérisation de l'objet sur la base des informations de positionnement réel ;
l'envoi de l'objet à la position cible pour la numérisation en réponse à la détermination que la dose de numérisation remplit une exigence.

7. Procédé selon l'une quelconque des revendications 1-6, dans lequel la pluralité de premières images inclut une vidéo du dispositif médical (110), le résultat de l'analyse inclut des informations de fonctionnement réel du dispositif médical (110) et la commande du dispositif médical (110) sur la base d'un résultat d'analyse inclut la commande d'un état de fonctionnement du dispositif médical (110) par :
la commande de l'état de fonctionnement du dispositif médical (110) sur la base des informations de fonctionnement réel et d'informations de fonctionnement attendu, dans lequel les informations de fonctionnement réel incluent des informations d'image et des informations sonores.

8. Procédé selon la revendication 7, incluant en outre :
l'obtention d'informations de fonctionnement actuel du dispositif médical (110) ; et
la détermination des informations de fonctionnement attendu du dispositif médical (110) sur la base des informations de fonctionnement actuel.

9. Procédé selon la revendication 8, dans lequel l'obtention d'informations de fonctionnement actuel du dispositif médical (110) inclut au moins une parmi :
la désignation d'informations de commande interne du dispositif médical (110) comme les informations de fonctionnement actuel, les informations de commande interne étant acquises sur la base d'un dispositif de commande connecté au dispositif médical (110) ; ou
la désignation d'informations de commande de clé du dispositif médical (110) comme les informations de fonctionnement actuel, les informations de commande de clé étant acquises sur la base d'un second dispositif de surveillance (160-2).

10. Procédé selon la revendication 9, dans lequel, lorsque les informations de commande de clé indiquent qu'une clé est bloquée, les informations de fonctionnement attendu correspondant à la clé bloquée et les informations de fonctionnement attendu correspondant à la clé débloquée sont cohérentes.

11. Procédé selon l'une quelconque des revendications 8-10, dans lequel la détermination des informations de fonctionnement attendu du dispositif médical (110) sur la base des informations de fonctionnement actuel inclut :
l'obtention d'une correspondance prédéterminée entre les informations de fonctionnement actuel et les informations de fonctionnement attendu ; et
la détermination des informations de fonctionnement attendu sur la base de la correspondance prédéterminée et des informations de fonctionnement actuel.

12. Procédé selon l'une quelconque des revendications 7-11, dans lequel la commande de l'état de fonctionnement du dispositif médical (110) sur la base des informations de fonctionnement réel et des informations de fonctionnement attendu inclut :
en réponse à la détermination que les informations de fonctionnement attendu sont incohérentes avec les informations de fonctionnement réel, la détermination que le dispositif médical (110) se trouve dans un état anormal ; et
l'envoi d'une instruction d'arrêt de mouvement au dispositif médical (110) via un dispositif de commande connecté au dispositif médical (110) ; et
la génération d'informations d'avertissement.

13. Dispositif médical (110), comprenant :
un numériseur configuré pour réaliser une numérisation médicale afin d'obtenir des données de numérisation d'une région de numérisation d'un objet ;
un dispositif d'acquisition d'images configuré pour obtenir une pluralité de premières images d'une salle de numérisation (101) ;
un processeur configuré pour obtenir un résultat d'analyse en analysant la pluralité de premières images et déterminer un mode de commande du dispositif médical (110) sur la base du résultat d'analyse, dans lequel chacune d'au moins une partie de la pluralité de premières images inclut l'objet, le résultat d'analyse inclut des informations de posture de l'objet,
le processeur est en outre configuré pour mettre en œuvre un mécanisme de commande pour commander le dispositif médical (110) sur la base du mode de commande, dans lequel le dispositif médical (110) inclut une table d'examen (116), le mode de commande du dispositif médical (110) inclut la commande du dispositif médical (110) pour ajuster une hauteur de la table d'examen (116) à une hauteur cible sur la base des informations de posture de l'objet, le dispositif médical étant **caractérisé en ce que** le processeur est en outre configuré pour déterminer les informations de posture comme une posture assise, une posture debout ou une posture couchée, et les informations de posture sont déterminées par :
la détermination de points caractéristiques d'un corps de l'objet sur la base de la pluralité de premières images ;
la détermination si l'objet est dans la posture couchée sur la base des informations des points caractéristiques ; et
la détermination si l'objet est dans la posture assise ou debout sur la base des articulations des jambes de l'objet lors de la détermination que le corps de l'objet est dans un état vertical sur la base des informations des points caractéristiques ;
et **en ce que** le mécanisme de commande mis en œuvre par le processeur inclut en outre :
en réponse à la détermination que l'objet entre dans la salle de numérisation (101), l'obtention d'une longueur d'une jambe de l'objet ; et l'abaissement de la hauteur de la table d'examen (116) lorsque l'objet se dirige vers la table d'examen (116) en dessous de la longueur de la jambe de l'objet ;
en réponse à la détermination que l'objet entre dans la salle de numérisation (101) avec un fauteuil roulant, l'obtention d'une hauteur du fauteuil roulant ; et l'abaissement de la hauteur de la table d'examen (116) lorsque l'objet se dirige vers la table d'examen (116) en dessous de la hauteur du fauteuil roulant ; ou
en réponse à la détermination que l'objet entre dans la salle de numérisation (101) sur une civière, l'obtention d'une hauteur de la civière et l'abaissement de la hauteur de la table d'examen (116) lorsque l'objet se dirige vers la table d'examen (116) en dessous de la hauteur de la civière.

14. Système de surveillance d'un dispositif médical (110), comprenant :
un module d'acquisition configuré pour obtenir une pluralité de premières images d'une salle de numérisation (101) acquises par un premier dispositif de surveillance (160-1) agencé dans la salle de numérisation (101) ;
un module d'analyse d'images configuré pour obtenir un résultat d'analyse en analysant une pluralité de premières images, chacune d'au moins une partie de la pluralité de premières images inclut un objet, le résultat d'analyse inclut des informations de posture de l'objet, un module de commande configuré pour commander le dispositif médical (110) sur la base du résultat d'analyse, le dispositif médical (110) incluant une table d'examen (116), le système étant **caractérisé en ce que** le module d'analyse d'images obtient un résultat d'analyse en tant que les informations de posture, incluant une posture assise, debout ou couchée, et les informations de posture sont déterminées par :
la détermination de points caractéristiques d'un corps de l'objet sur la base de la pluralité de premières images ;
la détermination si l'objet est dans la posture couchée sur la base des informations des points caractéristiques ; et
la détermination si l'objet est dans la posture assise ou debout sur la base des articulations des jambes de l'objet lors de la détermination que le corps de l'objet est dans un état vertical sur la base des informations des points caractéristiques ;
et dans lequel le module de commande met en œuvre un mode de commande du dispositif médical (110) qui inclut la commande du dispositif médical (110) pour ajuster une hauteur de la table d'examen (116) à une hauteur cible sur la base des informations de posture de l'objet, incluant :
en réponse à la détermination que l'objet entre dans la salle de numérisation (101), l'obtention d'une longueur d'une jambe de l'objet ; et l'abaissement de la hauteur de la table d'examen (116) lorsque l'objet se dirige vers la table d'examen (116) en dessous de la longueur de la jambe de l'objet ;
en réponse à la détermination que l'objet entre dans la salle de numérisation (101) avec un fauteuil roulant, l'obtention d'une hauteur du fauteuil roulant ; et l'abaissement de la hauteur de la table d'examen (116) lorsque l'objet se dirige vers la table d'examen (116) en dessous de la hauteur du fauteuil roulant ; ou
en réponse à la détermination que l'objet entre dans la salle de numérisation (101) sur une civière, l'obtention d'une hauteur de la civière et l'abaissement de la hauteur de la table d'examen (116) lorsque l'objet se dirige vers la table d'examen (116) en dessous de la hauteur de la civière.

15. Procédé selon la revendication 5, le procédé comprenant en outre :
avant l'envoi de l'objet à la position cible,
la détermination d'une région d'interférence correspondant à la table d'examen (116) sur la base de la table d'examen (116) et de la trajectoire de mouvement depuis une position où l'objet se dirige vers la table d'examen jusqu'à la position cible,
la détermination si une interférence se produit entre l'objet et un ou plusieurs composants du dispositif médical (110) sur la base de la région d'interférence ;
en réponse à l'absence d'interférence entre l'objet et le ou les composants, l'envoi de l'objet à la position cible pour une numérisation ; et
en réponse à l'interférence entre l'objet et le ou les composants, l'ajustement d'une posture de numérisation de l'objet afin d'éliminer tout risque d'interférence.
